# FASCICULE DE BREVET EUROPEEN

(11) **EP 1 747 213 B1**
(45) Date de publication et mention de la délivrance du brevet: **26.05.2010**
(21) Numéro de dépôt: 05771126.9
(22) Date de dépôt: 13.05.2005
(51) Int. Cl.: C07D 321/10, C07C 29/132

(54) **NOUVEAUX DERIVES 7,7-DISUBSTITUES DU (5H,9H)-6,8-DIOXABENZOCYCLOHEPTENE, LEUR PREPARATION ET LEUR UTILISATION DANS LA SYNTHESE D'ANALOGUES NON STEROIDIENS DE LA VITAMINE D**
NEUARTIGE 7,7-DISUBSTITUIERTE DERIVATE VON (5H,9H)-6,8-DIOXABENZOCYCLOHEPTEN, HERSTELLUNGSVERFAHREN DAFÜR UND IHRE VERWENDUNG IN DER SYNTHESE VON NICHT-STEROIDALEN VITAMIN-D-ANALOGEN
NOVEL 7,7-DISUBSTITUTED DERIVATIVES OF (5H,9H)-6,8-DIOXABENZOCYCLOHEPTENE, PREPARATION METHOD THEREOF AND USE OF SAME IN THE SYNTHESIS OF NON-STEROIDAL VITAMIN D ANALOGUES

(30) Priorité: 14.05.2004 FR 0405282; 07.06.2004 US 577201 P
(43) Date de publication de la demande: 31.01.2007
(73) Titulaire: Galderma Research & Development, 06410 Biot (FR)
(72) Inventeur: TERRANOVA, Eric, F-06520 Magagnosc (FR); PASCAL, Jean-Claude, F-06100 Nice (FR)
(74) Mandataire: Allab, Myriam
(86) Numéro de dépôt international: PCT/FR2005/001210
(87) Numéro de publication internationale: WO 2005/116007

(56) Documents cités:
- WO-A-01/38303
- WO-A-03/050067
- US-B1- 6 235 731

## Description

La présente invention concerne de nouveaux dérivés 7,7-disubstitués du (5*H,*9*H*)-6,8-dioxabenzocycloheptène, leurs procédés de préparation et leur utilisation dans la synthèse d'analogues non stéroïdiens de la vitamine D.

La présente invention concerne de nouveaux dérivés 7,7-disubstitués du (5*H,*9*H*)-6,8-dioxabenzocycloheptène de formule générale (I) : 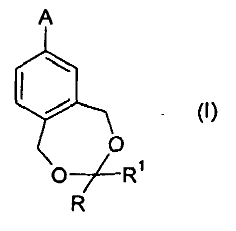 dans laquelle :
- A représente un groupement -CH₂-Y ou un groupement carboxaldéhyde -C(=O)H ;
- Y est choisi parmi un atome d'halogène, choisi parmi un atome de chlore ou de brome, un radical hydroxyle, un radical -OSO₂R², et un radical P(O)(OR³)₂ ;
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro ;
- R et R¹ pouvant également former ensemble et avec l'atome de carbone qui les porte un cycle de 5 ou 6 atomes de carbone ;
- R² représente un radical alkyle ou un radical aryle ; et
- R³ représente un radical alkyle ;
   et leurs éventuels isomères optiques.

En particulier, les composés de formule générale (I) seront tels que R et R¹ ne peuvent être simultanément un radical méthyle lorsque A est le radical -CH2OH.

Par radical alkyle, on entend un radical alkyle, linéaire ou ramifié, possédant de 1 à 4 atomes de carbone, et en particulier les radicaux méthyle, éthyle, propyle, isopropyle, 2-méthyle propyle, butyle, *sec*-butyle et *tert*-butyle.

De manière similaire, par radical alkoxy, on entend, un radical alkoxy, linéaire ou ramifié, possédant de 1 à 4 atomes de carbone, et en particulier les radicaux méthoxy, éthoxy, propoxy, isopropoxy, butoxy, *sec*-butoxy et *tert*-butoxy.

Par radical aryle, on entend phényle, naphtyle ou pyridyle.

Par cycle de 5 ou 6 atomes de carbone, on entend un radical cyclopentane et un radical cyclohexane.

Selon la présente invention, les composés préférés sont ceux pour lesquels A représente un groupement -CH2-Y, Y représente atome de chlore ou de brome ou un radical OSO₂R² ou un radical P(O)(OR³)₂, et R représente un atome d'hydrogène et R¹ représente un radical nitrophényle ou inversement pour R et R¹.

Selon la présente invention, les composés préférés sont également ceux pour lesquels A représente un groupement -CH2-Y, Y représente un radical hydroxyle, R représente un atome d'hydrogène et R¹ représente un radical nitrophényle ou inversement pour R et R¹.

Parmi les composés de formule (I), de façon préférée au moins l'un de R ou R¹ ne représente pas l'hydrogène.

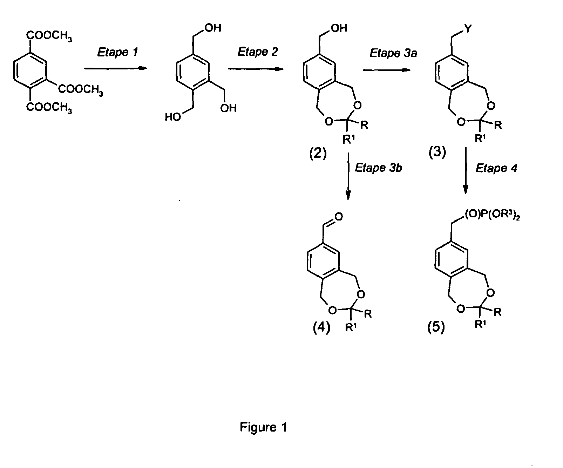

La présente invention concerne également le procédé de préparation des composés de formule générale (I) selon le schéma réactionnel suivant :
Selon le schéma réactionnel présenté en figure 1, les composés de formule générale (I) peuvent être préparés à partir d'un trialkyle ester de l'acide benzène-1,2,4-tricarboxylique selon les étapes suivantes :
   - en étape 1, un trialkyle ester de l'acide benzène-1,2,4-tricarboxylique, par exemple le benzène-1,2,4-tricarboxylate de triméthyle, est soumis à une réaction de réduction, comme décrit par exemple dans NAKAZAKI, M.; YAMAMOTO, K. et MIURA, Y. ; J. Org. Chem., 43(6), (1978), 1041-1044, par exemple au moyen de borohydrure de lithium, dans un solvant aprotique, tel que le tétrahydrofurane, pour conduire au (3,4-bis-hydroxyméthylphényl)méthanol,
   - en étape 2, ledit (3,4-bis-hydroxyméthylphényl)méthanol est lui-même soumis à une réaction de protection sélective des deux fonctions alcool benzylique adjacentes en positions 3 et 4 par réaction avec une cétone ou un aldéhyde de formule R-CO-R¹, , dans un solvant aprotique, comme par exemple le dichlorométhane, en présence d'un catalyseur, avantageusement de zéolite, telle la zéolite HZSM-5 ou la zéolite HY, comme décrit par exemple par Tajbakhsh, M. et coll. (Synth. Commun., (1999), 29(1), 135-138) ou encore par Kumar, T. P. et coll. (J. Chem. Res., Synop., (1994), 10, 394-395), ou la zéolithe EPZG, comme décrit par Bandgar, B. P. et coll. (Synth. Commun., (1997), 27(4), 627-634), ou encore en présence de quantités catalytiques de montmorillonite KSF, comme décrit par Patney, H. K. (Synth. Commun., (1993), 23(11), 1523-1526), pour conduire aux composés de formule générale (2) : 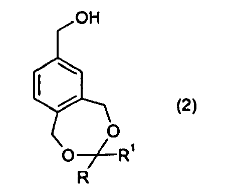
   - en étape 3(a), les composés de formule (2) ainsi obtenus peuvent ensuite, par réaction avec un dérivé R²SO₂Cl, être transformés en composés de formule (3) : 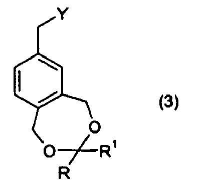 dans laquelle Y, selon les conditions réactionnelles, représente un atome d'halogène, choisi parmi un atome de chlore ou de brome, ou un groupement -OSO₂R²,
   - en étape 4, les composés de formule (3) ci-dessus définis et pour lesquels Y représente un atome d'halogène, choisi parmi un atome de chlore ou de brome, peuvent à leur tour être transformés en composés de formule (5) : 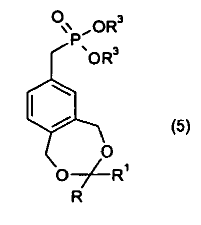 par réaction avec un dérivé de trialkylphosphite P(OR³)₃, selon un procédé similaire à celui décrit par Moreau, B. et coll. (Org. Prep. Proced. Int., (2002), 34(5), 539-542).

Selon le schéma réactionnel présenté en figure 1, les composés de formule générale (I) peuvent encore être préparés à partir d'un trialkyle ester de l'acide benzène-1,2,4-tricarboxylique selon les mêmes étapes 1 et 2,
et en étape 3(b,) les composés de formule (2) peuvent être engagés dans une réaction d'oxydation, par action d'un agent oxydant, tel que l'oxyde de manganèse ou le chlorochromate de pyridinium, pour conduire aux composés de formule (4) : 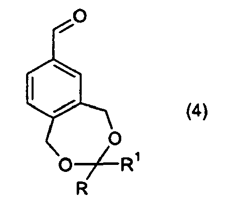 l'ensemble des composés de formules (2), (3), (4) et (5) représentant la totalité des composés de formule (I) précédemment définis et objets de l'invention.

Il est entendu, sauf indication contraire, dans ce tout qui précède que Y, R, R¹, R² et R³ sont tels que définis dans la formule (I).

Les matières premières utilisées pour la préparation des composés de formule (I) selon l'invention sont disponibles dans le commerce ou aisément accessibles à partir de matières premières commerciales, selon des procédés connus ou dérivés de procédés connus et connus de l'homme du métier ou aisément accessibles à partir de la littérature brevet ou d'articles scientifiques, des Chemical Abstracts et de l'Internet.

Selon un autre objet de la présente invention, les composés de formule générale (I) sont utiles comme intermédiaires de synthèse d'analogues non stéroïdiens de la vitamine D.

Dans la demande de brevet WO03/050067, il est décrit l'introduction de la partie mimant le cycle A des analogues non stéroïdiens de la vitamine D soit par couplage de l'ester diméthylique de l'acide 4-bromométhylphthalique, préparé en trois étapes à partir de l'anhydride trimellitique, soit par réaction du bromure de (3,4-bis-benzoyloxyméthyl)benzyle préparé en 7 étapes, également à partir de l'anhydride trimellitique, soit encore par réaction de l'ester diméthylique de l'acide 4-(diéthoxyphosphorylméthyl)phtalique, préparé en 4 étapes à partir de l'anhydride triméllitique. Dans les cas où l'on utilise un dérivé de l'acide phthalique, la dernière étape consiste en une réduction des deux fonctions ester.

Dans la demande de brevet WO01/38303, il est décrit la synthèse de dérivés de vitamines D. Toutefois, cette demande ne décrit pas, ni ne suggère, l'utilisation de composés de structure dioxabenzocycloheptene pour la préparation de composés de vitamine D.

Dans le brevet US6235731, il est décrit l'utilisation du composé (7,7-diméthyl-5,9-dihydro-6,8-dioxabenzocyclohepten-2-yl) méthanol pour réaliser la synthèse du composé N-[2-(3-phenylpropan-1-yl)-2,3-dihydro-1H-isoindol-5-ylmethyl] phtalimide (colonne 53, exemple 7, étape 1). Ce dernier, qui n'est pas un dérivé de vitamine D, est utilisé comme agent antihyperlipidémiant.

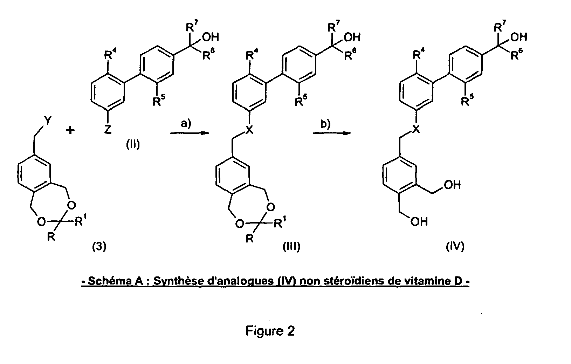

La présente invention a pour objet les procédés de préparation de nouveaux dérivés 7,7-disubstitués du (5*H,*9*H*)-6,8-dioxabenzocycloheptène de formule (I) qui présentent, l'avantage d'offrir une nouvelle méthode de synthèse des analogues non stéroïdiens de la vitamine D tels que ceux décrits dans la demande WO03/050067. De façon préférée, après couplage avec une partie biphényle, les composés de formule (I) permettent d'obtenir les composés de formule (III) tels que définis et présentés dans la figure 2, puis, en milieu acide dilué ou par hydrogénolyse en présence d'une quantité catalytique d'oxyde de platine ou de palladium permettent d'obtenir les composés de formule générale (IV), tels que définis ci-dessous.

Ces composés préparés en seulement trois ou quatre étapes constituent donc des intermédiaires nouveaux et utiles dans la synthèse d'analogues non stéroïdiens de la vitamine D.

En particulier, les composés de formule (I) de la présente invention offrent l'avantage d'obtenir des analogues non stéroïdiens de la vitamine D via un procédé comportant un nombre inférieur d'étapes par rapport aux procédés décrits dans l'art antérieur, et faisant intervenir des étapes simples et sélectives de protection et de déprotection.

Ainsi, la présente invention concerne également l'utilisation des dérivés 7,7-disubstitués du (5*H,*9*H*)-6,8dioxabenzocycloheptène de formule (I) telle que définie précédemment, et en particulier les composés (2), (3), (4) et (5) définis ci-dessus, comme intermédiaires de synthèse, pour la préparation de dérivés non stéroïdiens de vitamine D.

Préférentiellement, les composés de formules (I), en particulier les composés (2), (3), (4) et (5) selon l'invention sont utilisés comme intermédiaire de synthèse de dérivés non stéroïdiens de vitamine D. Dans la suite du présent exposé, il sera compris que la formule (I) englobe tous les composés de formules (2), (3), (4) et (5) et que les composés de formules (2), (3), (4) et (5) correspondent aux composés de formule (I).

Plus préférentiellement, les composés de formules (I), en particulier les composés (2), (3), (4) et (5) selon l'invention sont utilisés comme intermédiaire de synthèse de dérivés non stéroïdiens de vitamine D répondant à la formule (IV) suivante : 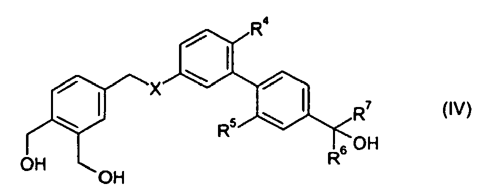 dans laquelle
R⁴ et R⁵, identiques ou différents, représentent indépendamment les uns des autres un radical alkyle linéaire ou ramifié comportant de 1 à 3 atomes de carbone ;
R⁶ et R⁷, identiques, sont choisis, parmi un radical alkyle linéaire ou ramifié comportant de 1 à 2 atomes de carbone, hydrogéné ou perfluoré;
X représente -CH₂-, -NH-, -NR³- ou -O- et
R⁸ représente un radical alkyle de 1 à 3 atomes de carbone linéaire ou ramifié.

Par radical alkyle comportant de 1 à 3 atomes de carbone selon la présente invention on entend un radical méthyle, un radical éthyle, un radical propyle ou un radical isopropyle.

Par radical alkyle comportant de 1 à 2 atomes de carbone, hydrogéné ou perfluoré, selon la présente invention on entend un radical éthyle ou un radical trifluorométhyle.

Selon une variante préférée de la présente invention, les composés de formule (3) selon l'invention interviennent dans la synthèse d'analogues non stéroïdiens de la vitamine D, qui répondent à la formule (IV) telle que définie précédemment, selon le schéma A présenté en figure 2,
schéma dans lequel Y représente un atome d'halogène, choisi parmi un atome de chlore ou de brome, ou un radical -OSO₂R², Z représente un radical hydroxyle, ou un radical -NHR⁸, R⁸ représentant un radical alkyle linéaire ou ramifié de 1 à 3 atomes de carbone, R, R¹, R⁴, R⁵, R⁶, R⁷ et X sont tels que définis précédemment,
l'étape a) correspondant à une réaction de couplage du composé de formule (3) de la présente invention : 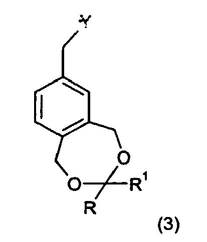 avec un composé de formule (II) : 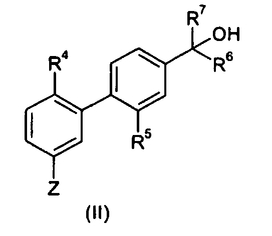 c'est-à-dire par réaction de couplage entre les deux fonctions réactives -CH₂-Y- et Z conduisant à la formation d'une liaison -CH₂-X-, Y et X étant tels que définis précédemment, pour l'obtention d'un composé de formule (III) : 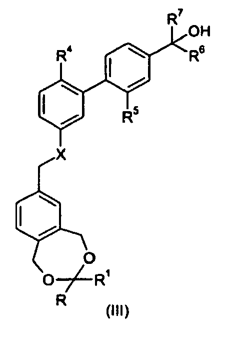 l'étape b) correspondant à une réaction classique de dé-protection des deux fonctions alcools benzyliques adjacentes en positions 3 et 4 contenues dans le cycle (5*H,*9*H*)-6,8-dioxabenzocycloheptène du composé de formule (III), ledit cycle provenant du composé de formule (3), pour l'obtention du composé de formule (IV) :

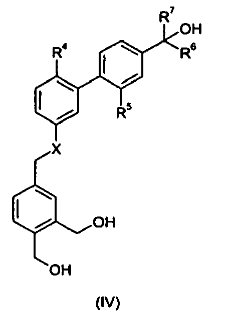

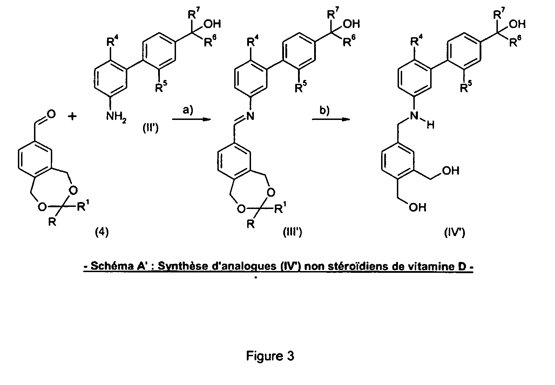

Selon une autre variante préférée de la présente invention, les analogues non stéroïdiens de formule (IV'), analogues de formule (IV) dans lesquels X représente -NH-, peuvent avantageusement être préparés à partir des composé de formule (4) selon l'invention et des composés de formule (II'), c'est-à-dire des composés de formule (II) où Z représente -NH₂, selon le schéma A' présenté en figure 3,
schéma dans lequel R, R¹, R⁴, R⁵, R⁶ et R⁷ sont tels que définis précédemment,
l'étape a) correspondant à une réaction classique de couplage entre la fonction aldéhyde du composé de formule (4) : 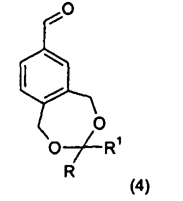 et la fonction amine du composé de formule (II') : 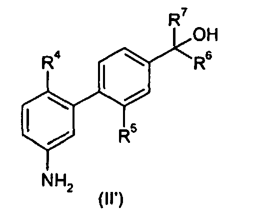 pour l'obtention du composé de formule (III') : 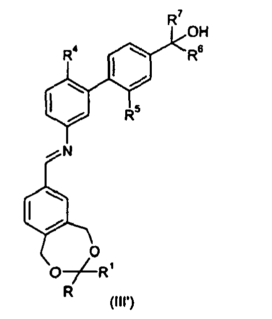 l'étape b) étant une réaction d'hydrogénation et d'hydrogénolyse sur le composé de formule (III'), permettant, en une seule étape, à la fois la réduction de la fonction imine (hydrogénation) et la dé-protection des deux fonctions alcools benzyliques adjacentes en positions 3 et 4 contenues dans le cycle (5*H,*9*H*)-6,8-dioxabenzocycloheptène provenant du composé de formule (4) (hydrogénolyse), pour l'obtention du composé de formule (IV') :

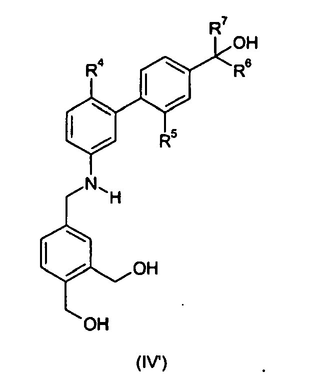

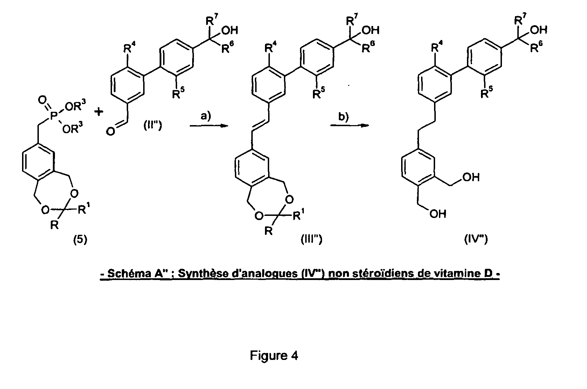

Selon une autre variante préférée de la présente invention, les analogues non stéroïdiens de formule (IV"), analogues de formule (IV) dans lesquels X représente -CH₂-, peuvent avantageusement être préparés à partir des composé de formule (5) selon l'invention et des composés de formule (II"), c'est-à-dire des composés de formule (II) où Z représente un groupement carboxaldéhyde -C(=O)H, selon le schéma A" présenté en figure 4 :
schéma dans lequel R, R¹, R³, R⁴, R⁵, R⁶ et R⁷ sont tels que définis précédemment,
   l'étape a) correspondant à une réaction classique dite de Horner-Emmons entre le composé de formule (5) : 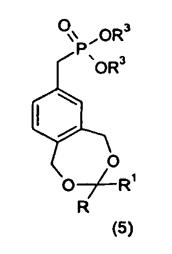 et le composé de formule (II") : 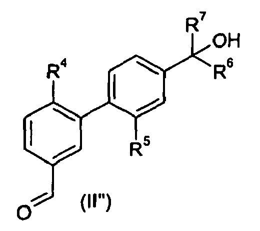 pour l'obtention du composé de formule (III") : 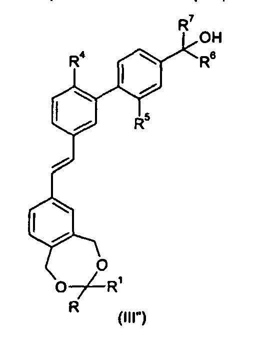 l'étape b) étant une réaction d'hydrogénation et d'hydrogénolyse sur le composé de formule (III") permettant, en une seule étape, à la fois la réduction de la double liaison (hydrogénation) et la dé-protection des deux fonctions alcools benzyliques adjacentes en positions 3 et 4 contenues dans le cycle (5*H,*9*H*)-6,8-dioxabenzocycloheptène provenant du composé de formule (5) (hydrogénolyse), pour l'obtention du composé (VI") :

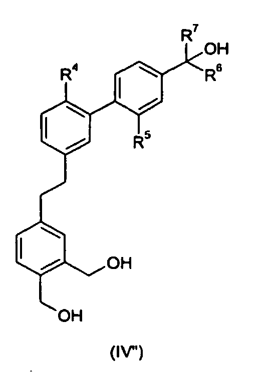

La présente invention se rapporte donc aussi à l'utilisation des composés de formule générale (II) : 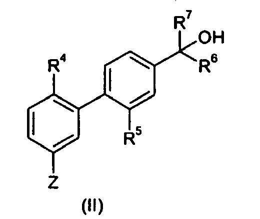 pour la préparation d'analogues non stéroïdiens de vitamine D de formule générale (IV) : 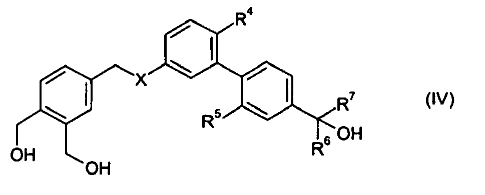 où
- R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis, parmi un radical alkyle linéaire ou ramifié comportant de 1 à 2 atomes de carbone, hydrogéné ou perfluoré ;
- X représente -CH₂-, -NH-, -NR⁸- ou -O- et
- R⁸ représente un radical alkyle de 1 à 3 atomes de carbone linéaire ou ramifié.

Selon un autre de ses objets, la présente invention se rapporte à l'utilisation des composés de formule générale (II') : 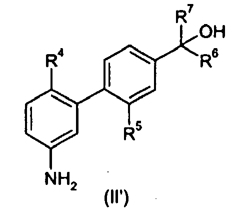 pour la préparation d'analogues non stéroïdiens de vitamine D de formule générale (IV') : 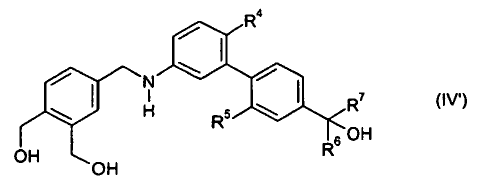 où
- R⁴ et R⁵, identiques ou différents, représentent indépendamment les uns des autres un radical alkyle linéaire ou ramifié comportant de 1 à 3 atomes de carbone :
- R⁶ et R⁷, identiques, sont choisis, parmi un radical alkyle linéaire ou ramifié comportant de 1 à 2 atomes de carbone, hydrogéné ou perfluoré.

Enfin, la présente invention se rapporte également à l'utilisation des composés de formule générale (II") 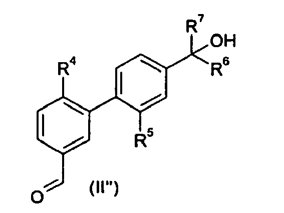 pour la préparation d'analogues non stéroïdiens de vitamine D de formule générale (IV") : 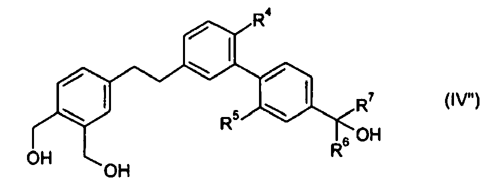 où
R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis parmi un radical alkyle comportant de 1 à 2 atomes de carbone, hydrogéné ou perfluoré.

Les exemples suivants illustrent la présente invention sans la limiter en aucune façon.

### Exemples :

### Exemple 1 : Préparation du [7-(4-nitrophényl)-(5H,9H)-6,8-dioxabenzocycloheptén-2-yl]méthanol

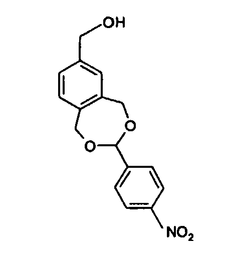

Dans un ballon équipé d'un Dean Stark, on introduit 10 g de (3,4-bis-hydroxyméthylphényl)méthanol (59,4 mmoles) (préparé selon NAKAZAKI, M. ; YAMAMOTO, K. et MIURA, Y. ; J. Org. Chem., 43(6), (1978), 1041-1044), 100 mL de toluène, 8,98 g de 4-nitrobenzaldéhyde (59,4 mmoles) et 0,6 g de Montmorillonite KSF (10 mg / mmole). On chauffe au reflux pendant 2 heures 30 minutes. On filtre la Montmorillonite et on laisse revenir le milieu réactionnel à température ambiante sous agitation. Le produit de réaction cristallise. Après filtration et séchage sous vide à 50°C, on obtient 11,2 g de [7-(4-nitrophényl)-(5*H,*9*H*)-6,8-dioxabenzocyclo-heptén-2-yl]méthanol.

Rendement = 58%

Point de fusion = 146°C

RMN ¹H (DMSO d₆ 400,132 MHz ) δ (ppm) : 4,49 (d ; 2H ; J=5,6Hz) ; 4,91 (d; 2H ; J=14,1Hz) ; 5,13 (dd ; 2H ; J=5,7Hz et J=14,1Hz) ; 5,20 (t ; 1H ; J=5,6Hz) ; 6,12 (s ; 1H) ; 7.24 (m ; 3H) ; 7,73 (d ; 2H ; J=8,7Hz) ; 8,24 (dd ; 2H ; J=1,9Hz et J=7,0Hz).

### Exemple 2 : Préparation du (7,7-spirocyclohexyl-(5H,9H)-6,8-dioxabenzocycloheptén-2-yl)méthanol

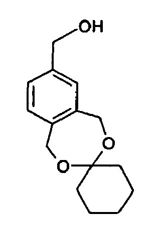

Dans un ballon équipé d'un Dean Stark, on introduit 4 g de (3,4-bis-hydroxyméthylphényl)méthanol (23,8 mmoles), 50 mL de toluène. 2,57 g de cyclohexanone (26,2 mmoles) et 0,24 g de Montmorillonite KSF (10 mg / mmole). On chauffe le milieu réactionnel au reflux pendant 2 heures 30 minutes. Après filtration de la Montmorillonite, on évapore le toluène sous vide à 30°C. On obtient 5,4 g du produit brut attendu. Après chromatographie sur gel de silice (heptane/acétate d'éthyle = 3/2), on obtient 1,3 g de (7,7-spirocyclohexyl-(5*H,*9*H*)-6,8-dioxabenzocycloheptén-2-yl)-méthanol.

Rendement = 22%

Point de fusion = 96°-98°C

RMN ¹H (DMSO d₆ ; 400,132 MHz) δ (ppm) : 1,40 (m ; 2H) ; 1,50 (m ; 4H) ; 1,72 (m ; 4H) ; 4,44 (d ; 2H ; J=5,7Hz) ; 4,77 (s ; 2H) ; 4,78 (s ; 2H) ; 5,13 (t ; 1H ; J=5,7Hz) ; 7,06 (m ; 3H).

### Exemple 3 : Préparation du (7-méthoxy-(5H,9H)-6,8-dioxabenzocycloheptén-2-yl)méthanol

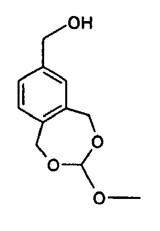

Dans un ballon, on introduit 4 g de (3,4-bis-hydroxyméthylphényl)méthanol (23,8 mmoles), 40 mL de diméthoxy-1,2-éthane, 2,78 g d'orthoformiate de méthyle (26,2 mmoles) et 0,24 g de Montmorillonite KSF (10 mg / mmole). On agite le milieu réactionnel à température ambiante pendant 4 heures 30 minutes puis on filtre la Montmorillonite et on évapore le diméthoxy-1,2-éthane sous vide à 30°C. Le résidu est chromatographié sur gel de silice (dichlorométhane/méthanol = 98/2), pour obtenir 3,2 g de (7-méthoxy-(5*H,*9*H*)-6,8-dioxabenzocycloheptén-2-yl)méthanol sous forme d'une huile.

Rendement = 64%

RMN ¹H (DMSO d₆ 400,132 MHz) δ (ppm) : 3,35 (s ; 3H) ; 4,46 (d ; 2H ; J=5,7Hz) ; 4,66 (d ; 2H ; J=14,9Hz) ; 4,94 (dd ; 2H ; J=5,9Hz et J=14,8Hz) ; 5,16 (t ; 1H ; J=5,7Hz) ; 7,13 (m ; 3H).

### Exemple 4 : Préparation du (7-phényl-(5H,9H)-6,8-dioxabenzocycloheptén-2-yl)-méthanol

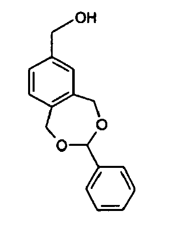

Dans un ballon équipé d'un Dean Stark, on introduit 4 g de (3,4-bis-hydroxyméthylphényl)méthanol (23,8 mmoles), 40 mL de toluène, 3,06 g de benzaldéhyde (28,5 mmoles) et 0,24 g de Montmorillonite KSF (10 mg / mmole). On porte le milieu au reflux pendant 3 heures. Après filtration le toluène est évaporé sous vide à 30°C. Le résidu est chromatographié sur gel de silice (heptane/acétate d'éthyle = 7/3), pour obtenir 1,8 g de (7-phényl-(5*H,*9*H*)-6,8-dioxabenzocycloheptén-2-yl)méthanol sous forme d'une poudre blanche.

Rendement = 30%

Point de fusion = 113°-114°C

RMN ¹H (DMSO d₆ 400,132 MHz) δ (ppm) : 4,49 (d ; 2H ; J=5,6Hz) ; 4,87 (d ; 2H ; J=14,1Hz) ; 5,05 (dd ; 2H ; J=5,2Hz et J=14,1Hz) ; 5,18 (t ; 1H ; J=5,6Hz) ; 5,97 (s ; 1H) ; 7,21 (m ; 3H) ; 7,38 (m ; 3H) ; 7,45 (m ; 2H).

### Exemple 5 : Préparation du (7,7-diméthyl-(5H,9H)-6,8-dioxabenzocycloheptén-2-yl)-méthanol

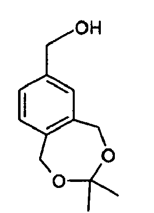

Dans un ballon, on introduit 5 g de (3,4-bis-hydroxyméthylphényl)méthanol (29,7 mmoles), 25 mL de 2,2-diméthoxypropane (21,2 g ; 0,2 moles) et 0,29 g d'acide p-toluènesulfonique (1,5 mmoles). Après 1 heure 10 minutes d'agitation à température ambiante, on évapore le solvant sous vide à 30°C et le produit brut est chromatographié sur gel de silice (dichlorométhane/méthanol/ammoniaque=98/2/0,1). On obtient 5,98 g de (7,7-diméthyl-(5*H*,9*H*)-6,8-dioxabenzocycloheptén-2-yl)méthanol sous forme d'une poudre blanche.

Rendement : 96%

RMN ¹H (CDCl₃; 400,132 MHz) δ (ppm) : 1,52 (s ; 6H) ; 4,6 (s ; 2H) ; 4,87 (s ; 4H) ; 7,08 (m ; 2H) ; 7,18 (m; 1H).

### Exemple 6 : Préparation du 2-chlorométhyl-7-(4-nitrophényl)-(5H,9H)-6,8-dioxabenzocycloheptène

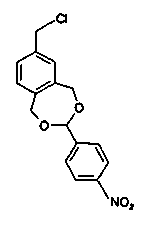

Dans un ballon, on introduit 1,5 g (5 mmoles) de [7-(4-nitrophényl)-(5*H,*9*H*)-6,8-dioxabenzocycloheptén-2-yl]méthanol dans 15 mL de dichlorométhane. On additionne 0,76 g (7,5 mmoles) de triéthylamine et on additionne par petites portions solides à température ambiante 1 g (5,4 mmoles) de chlorure de p-toluènesulfonyle. Après 20 heures de réaction, on évapore le solvant et on purifie le produit brut par chromatographie sur gel de silice (heptane/acétate d'éthyle : 4/1) pour obtenir 0,6 g de 2-chlorométhyl-7-(4-nitrophényl)-(5*H,*9*H*)-6,8-dioxabenzocycloheptène.

Rendement : 38%

Point de fusion : 138°-140°C

RMN ¹H (DMSO d₆ 400,132 MHz) δ (ppm) : 4,76 (s ; 2H) ; 4,93 (d ; 2H ; J=14,2Hz) ; 5,14 (d ; 2H ; J=14,2Hz) ; 6,14 (s ; 1H) ; 7,34 (m ; 3H) ; 7,73 (d ; 2H ; J=8,7Hz) ; 8,25 (d : 2H ; J=8,7Hz).

### Exemple 7 : Préparation de l'acide méthanesulfonique de [7-(4-nitrophényl)-(5H,9H)-6,8-dioxabenzocycloheptén-2-yl]méthyle ester

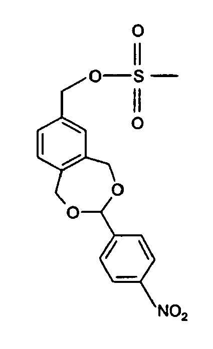

Dans un ballon, on introduit 3 g (10 mmoles) de [7-(4-nitrophényl)-(5*H,*9*H*)-6,8-dioxabenzocycloheptén-2-yl]-méthanol dans 50 mL de dichlorométhane. On additionne 1,54 g (12 mmoles) de diisopropyléthylamine et on additionne, goutte à goutte à -5°C, 1,37 g (12 mmoles) de chlorure de mésyle en solution dans 10 mL de dichlorométhane. Après 1 heure 30 minutes de réaction à -5°C, on additionne 20 mL d'une solution de bicarbonate de sodium à 5%, on décante les phases et on lave la phase organique à l'eau. On la sèche sur sulfate de sodium et on évapore le solvant. On purifie le produit brut par chromatographie sur gel de silice (heptane/acétate d'éthyle : 3/2) pour obtenir 0,6 g de l'acide méthanesulfonique de [7-(4-nitrophényl)-(5*H,*9*H*)-6,8-dioxabenzocycloheptén-2-yl]méthyle ester.

Rendement : 16%.

RMN ¹H (CDCl₃; 400,132 MHz) δ (ppm) : 2,97 (s ; 3H) ; 5,01 (m ; 4H) ; 5,24 (s ; 2H) ; 6,01 (s ; 1H) ; 7,26 (m ; 2H) ; 7,33 (dd ; 1H ; J=1,4Hz et J=7,6Hz) ; 7,77 (d ; 2H ; J=8,6Hz) ; 8,26 (dd ; 2H ; J=1,8Hz et J=7,0Hz).

## Revendications

1. Composé 7,7-disubstitués du (5*H*,9*H*)-6,8-dioxabenzocyclohepténe de formule générale (I) : 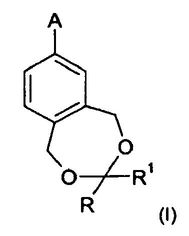 dans laquelle :
- A représente un groupement -CH₂-Y ou un groupement carboxaldéhyde -C(=O)H ;
- Y est choisi parmi un atome d'halogène, tel que le chlore ou le brome, un radical hydroxyle, un radical -OSO₂R², et un radical P(O)(OR³)₂ ;
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro ;
- R et R¹ pouvant également former ensemble et avec l'atome de carbone qui les porte un cycle de 5 ou 6 atomes de carbone ;
- R² représente un radical alkyle, ou un radical aryle ; et
- R³ représente un radical alkyle;
étant entendu que R et R¹ ne peuvent être simultanément un radical méthyle lorsque A est le radical -CH2OH
et leurs isomères optiques.

2. Composé selon la revendication 1, **caractérisé en ce que** au moins l'un de R ou R¹ ne représente pas l'hydrogène.

3. Composé selon la revendication 1 ou 2, **caractérisé en ce que** les radicaux alkyles ayant de 1 à 4 atomes de carbone sont choisis parmi les radicaux méthyle, éthyle, propyle, isopropyle, 2-méthyle propyle, butyle, *sec*-butyle et *tert*-butyle.

4. Composé selon l'une quelconque des revendications précédentes, **caractérisés en ce que** les radicaux alkoxy, sont choisis parmi les radicaux méthoxy, éthoxy, propoxy, isopropoxy, butoxy, *sec*-butoxy et *tert*-butoxy.

5. Composé selon l'une quelconque des revendications précédentes, **caractérisé en ce que** les radicaux aryle sont choisis parmi phényle, naphtyle et pyridyle.

6. Composé selon l'une quelconque des revendications précédentes, **caractérisé en ce que** R et R¹ forment ensemble et avec l'atome de carbone qui les porte un cycle de 5 ou 6 atomes de carbone choisi parmi un radical cyclopentane et un radical cyclohexane.

7. Composé selon l'une quelconque des revendications 1 à 5, **caractérisé en ce que** A représente un groupement -CH₂-Y, Y représente un atome de chlore ou de brome ou un radical OSO₂R² ou un radical P(O)(OR³)₂, et R représente un atome d'hydrogène et R¹ représente un radical nitrophényle ou inversement pour R et R¹.

8. Composé selon l'une quelconque des revendications 1 à 5, **caractérisé en ce que** A représente un groupement -CH₂-Y, Y représente un radical hydroxyle, et R représente un atome d'hydrogène et R¹ représente un radical nitrophényle ou inversement pour R et R¹.

9. Procédé de préparation des composés de formule (I) : 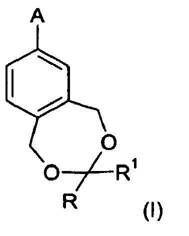 où
- A représente un groupement -CH₂-Y ou un groupement carboxaldéhyde -C(=O)H ;
- Y est choisi parmi un atome d'halogène, tel que le chlore ou le brome, un radical hydroxyle, un radical -OSO₂R², et un radical P(O)(OR³)₂ ;
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro ;
- R et R¹ pouvant également former ensemble et avec l'atome de carbone qui les porte un cycle de 5 ou 6 atomes de carbone ;
- R² représente un radical alkyle, ou un radical aryle : et
- R³ représente un radical alkyle;
**caractérisé en ce que** :
- en étape 1, un trialkyle ester de l'acide benzène-1,2,4-tricarboxylique est soumis à une réaction de réduction, dans un solvant aprotique, pour conduire au (3,4-bis-hydroxyméthylphényl)méthanol,
- en étape 2, ledit (3,4-bis-hydroxyméthylphényl)méthanol est soumis à une réaction de protection sélective des deux fonctions alcool benzylique adjacentes en positions 3 et 4 par réaction avec une cétone ou un aldéhyde de formule R-CO-R¹ dans un solvant aprotique, en présence d'un catalyseur, pour conduire aux composés de formule générale (2) : 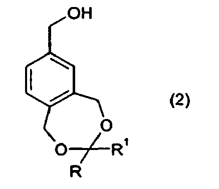
- en étape 3(a), les composés de formule (2) sont transformés, par réaction avec un dérivé R²SO₂Cl, en composés de formule (3) : 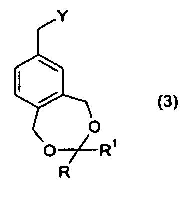
où Y représente un atome d'halogène, choisi parmi un atome de chlore ou de brome, ou un groupement -OSO₂R²,
- en étape 4, les composés de formule (3) sont transformés en composés de formule (5) : 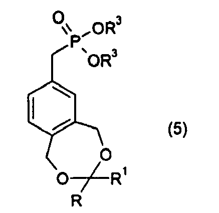 par réaction avec un dérivé de trialkylphosphite P(OR³)₃.

10. Procédé de préparation des composés de formule (I) : 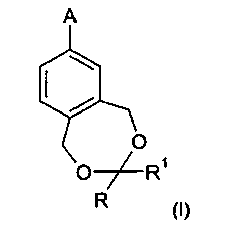 où
- A représente un groupement -CH₂-Y ou un groupement carboxaldéhyde -C(=O)H ;
- Y est choisi parmi un atome d'halogène, tel que le chlore ou le brome, un radical hydroxyle, un radical -OSO₂R², et un radical P(O)(OR³)₂ ;
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro ;
- R et R¹ pouvant également former ensemble et avec l'atome de carbone qui les porte un cycle de 5 ou 6 atomes de carbone ;
- R² représente un radical alkyle, ou un radical aryle ; et
- R³ représente un radical alkyle;
**caractérisé en ce que** :
- en étape 1, un trialkyle ester de l'acide benzène-1,2,4-tricarboxylique est soumis à une réaction de réduction, dans un solvant aprotique, pour conduire au (3,4-bis-hydroxyméthylphényl)méthanol ;
- en étape 2, ledit (3,4-bis-hydroxyméthylphényl)méthanol est soumis à une réaction de protection sélective des deux fonctions alcool benzylique adjacentes en positions 3 et 4 par réaction avec une cétone ou un aldéhyde de formule R-CO-R¹ dans un solvant aprotique, en présence d'un catalyseur, pour conduire aux composés de formule générale (2) : 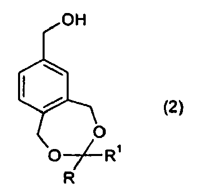
- en étape 3(b), les composés de formule (2) sont engagés dans une réaction d'oxydation, par action d'un agent oxydant, pour conduire aux composés de formule (4):

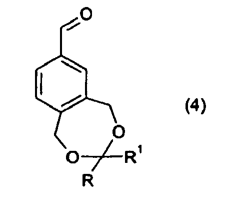

11. Composé de formule (2) suivante : 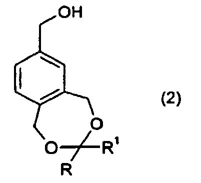 dans laquelle R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro, R et R¹ pouvant également former ensemble et avec l'atome de carbone qui les porte un cycle de 5 ou 6 atomes de carbone avec la condition que R et R¹ ne soient pas simultanément un radical méthyle.

12. Composés de formule (3) suivante : 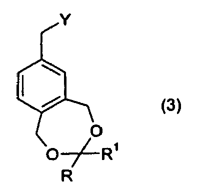 dans laquelle :
- Y représente un atome d'halogène, choisi parmi le chlore ou le brome, ou un groupement -OSO₂R²,
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro, R et R¹ pouvant également former ensemble et avec l'atome de carbone qui les porte un cycle de 5 ou 6 atomes de carbone et
- R² représente un radical alkyle, ou un radical aryle.

13. Composés de formule (4) suivante : 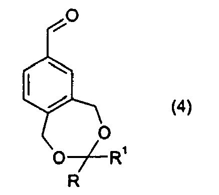 dans laquelle R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro, R et R¹ pouvant également former ensemble et avec l'atome de carbone qui les porte un cycle de 5 ou 6 atomes de carbone.

14. Composés de formule (5) suivante : 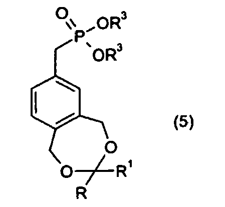 dans laquelle R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro, R et R¹ pouvant également former ensemble et avec l'atome de carbone qui les porte un cycle de 5 ou 6 atomes de carbone et R³ représente un radical alkyle.

15. Utilisation des composés de formule générale (I) : 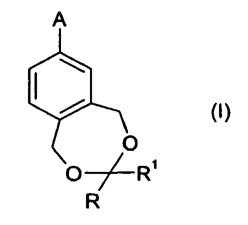 dans laquelle :
- A représente un groupement -CH₂-Y ou un groupement carboxaldéhyde -C(=O)H ; .
- Y est choisi parmi un atome d'halogène, choisi parmi le chlore ou le brome, un radical hydroxyle, un radical -OSO₂R², et un radical P(O)(OR³)₂ ;
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro ;
- R et R¹ pouvant également former ensemble et avec l'atome de carbone qui les porte un cycle de 5 ou 6 atomes de carbone ;
- R² représente un radical alkyle ou un radical aryle ; et
- R³ représente un radical alkyle ;
leurs éventuels isomères optiques,
pour la synthèse d'analogues non stéroïdiens de vitamine D.

16. Utilisation selon la revendication 15, **caractérisée en ce que** le composé de formule générale (I) est un composé de formule (2) suivante :

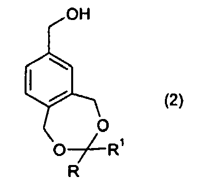

17. Utilisation selon la revendication 15, **caractérisée en ce que** le composé de formule générale (I) est un composé de formule (3) suivante : 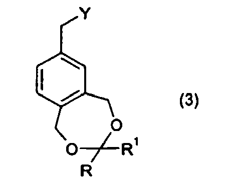 dans laquelle Y représente un atome d'halogène, choisi parmi le chlore ou le brome, ou un groupement -OSO₂R².

18. Utilisation selon la revendication 15, **caractérisée en ce que** le composé de formule générale (I) est un composé de formule (4) suivante :

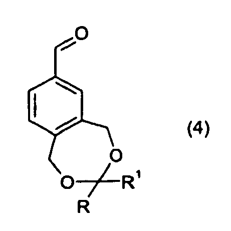

19. Utilisation selon la revendication 15, **caractérisée en ce que** le composé de formule générale (I) est un composé de formule (5) suivante :

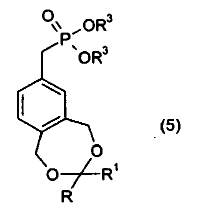

20. Utilisation selon l'une quelconque des revendications 15 à 19 pour la synthèse d'analogues non stéroïdiens de vitamine D de formule (IV) suivante : 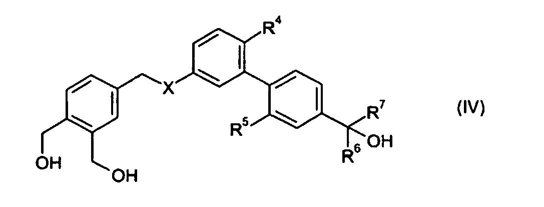 dans laquelle :
- R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis, parmi un radical alkyle linéaire ou ramifié comportant de 1 à 2 atomes de carbone, hydrogéné ou perfluoré ;
- X représente -CH₂-, -NH-, -NR⁸- ou -O- et
- R⁸ représente un radical alkyle de 1 à 3 atomes de carbone linéaire ou ramifié.

21. Procédé de synthèse d'analogues non stéroïdiens de la vitamine D, **caractérisé en ce qu'**au moins une étape utilise les composés de formule générale (I) suivante : 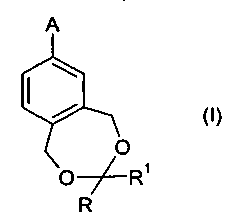 dans laquelle :
- A représente un groupement -CH₂-Y ou un groupement carboxaldéhyde -C(=O)H ;
- Y est choisi parmi un atome d'halogène, choisi parmi le chlore ou le brome, un radical hydroxyle, un radical -OSO₂R², et un radical P(O)(OR³)₂ ;
- R et R¹, identiques ou différents, sont choisis indépendamment l'un de l'autre parmi un atome d'hydrogène, un radical alkyle, un radical alkoxy, un radical aryle éventuellement substitué par un, deux, trois, quatre ou cinq substituants choisis parmi un atome d'halogène, l'halogène étant choisi parmi un atome de chlore, de brome, d'iode ou de fluor, un radical alkyle, un radical alkoxy, un cyano et un nitro ;
- R et R¹ pouvant également former ensemble et avec l'atome de carbone qui les porte un cycle de 5 ou 6 atomes de carbone ;
- R₂ représente un radical alkyle, ou un radical aryle ; et
- R₃ représente un radical alkyle ;
ainsi que leurs isomères optiques.

22. Procédé selon la revendication 21, **caractérisé en ce que** les analogues non stéroïdiens de la vitamine D sont les composés de formule générale (IV) suivante : 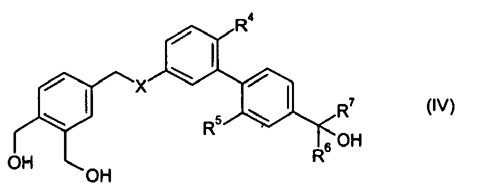 dans laquelle
- R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis parmi un radical alkyle comportant de 1 à 2 atomes de carbone, hydrogéné ou perfluoré ;
- X représente -CH₂-, -NH-, -NR⁸- ou -O- et
- R⁸ représente un radical alkyle linéaire ou ramifié de 1 à 3 atomes de carbone.

23. Procédé selon l'une quelconque des revendications 21 et 22, **caractérisé en ce qu'**il comprend les étapes suivantes :
a) réaction de couplage du composé de formule (3) de la présente invention : 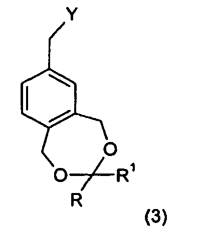 avec un composé de formule (II) : 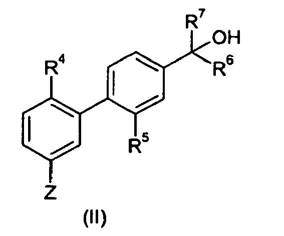 pour l'obtention d'un composé de formule (III) : 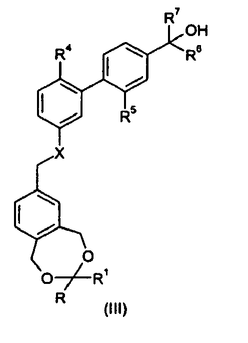
b) réaction de dé-protection des deux fonctions alcools benzyliques adjacentes en positions 3 et 4 contenues dans le cycle (5*H,*9*H*)-6,8-dioxabenzocycloheptène du composé de formule (III), ledit cycle provenant du composé de formule (3), pour l'obtention du composé de formule (IV) : 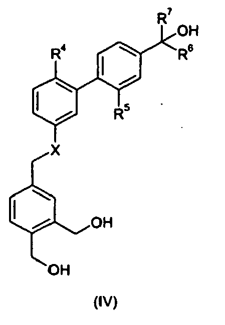 et dans lequel Y représente un atome d'halogène, choisi parmi un atome de chlore ou de brome, ou un radical -OSO₂R², Z représente un radical hydroxyle, ou un radical -NHR⁸, R⁸ représentant un radical alkyle linéaire ou ramifié de 1 à 3 atomes de carbone.

24. Procédé selon l'une quelconque des revendications 21 et 22, **caractérisée en ce qu'**il comprend les étapes suivantes :
a) réaction de couplage entre la fonction aldéhyde du composé de formule (4) : 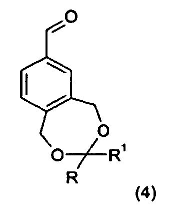 et la fonction amine du composé de formule (II') : 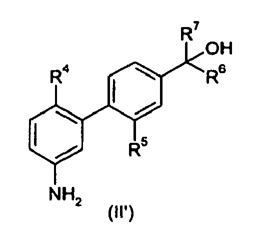 pour l'obtention du composé de formule (III') : 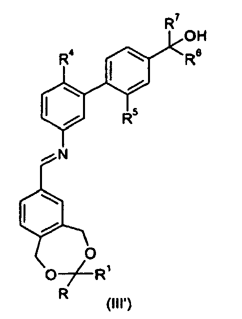
b) réaction d'hydrogénation et d'hydrogénolyse sur le composé de formule (III'), conduisant à la réduction de la fonction imine et la dé-protection des deux fonctions alcools benzyliques adjacentes en positions 3 et 4 contenues dans le cycle (5*H,*9*H*)-6,8-dioxabenzocycloheptène provenant du composé de formule (4) (hydrogénolyse), pour l'obtention du composé de formule (IV') :

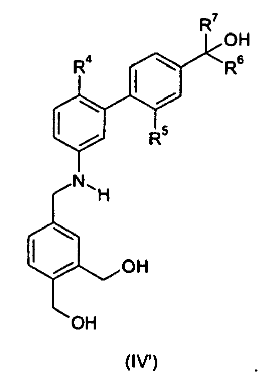

25. Procédé selon l'une quelconque des revendications 21 et 22, **caractérisé en ce qu'**il comprend les étapes suivantes :
a) une réaction dite de Horner-Emmons entre le composé de formule (5) : 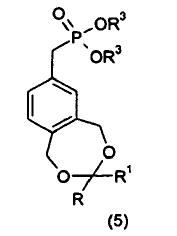 et le composé de formule (II") : 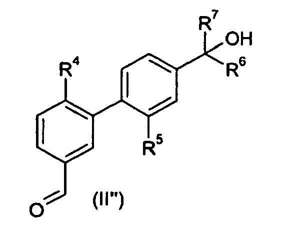 pour l'obtention du composé de formule (III") : 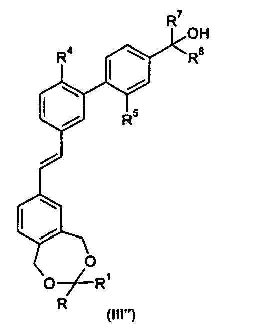
b) réaction d'hydrogénation et d'hydrogénolyse sur le composé de formule (III") conduisant à la réduction de la double liaison (hydrogénation) et la dé-protection des deux fonctions alcools benzyliques adjacentes en positions 3 et 4 contenues dans le cycle (5*H,*9*H*)-6,8-dioxabenzocycloheptène provenant du composé de formule (5) (hydrogénolyse), pour l'obtention du composé (VI") :

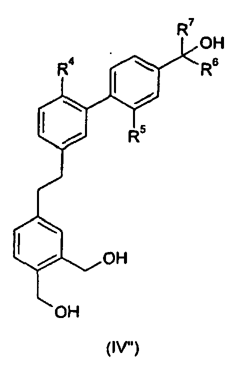

26. Utilisation des composés de formule générale (II) : 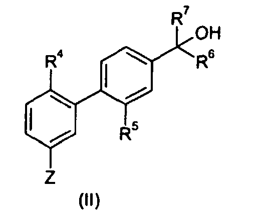 pour la préparation d'analogues non stéroïdiens de vitamine D de formule générale (IV) : 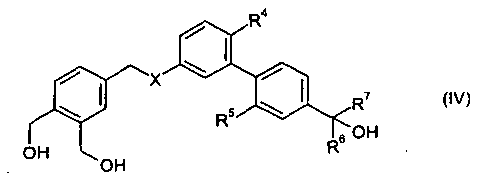 où
- R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis, parmi un radical alkyle linéaire ou ramifié comportant de 1 à 2 atomes de carbone, hydrogéné ou perfluoré ;
- X représente -CH₂-, -NH-, -NR⁸- ou -O- et
- R⁸ représente un radical alkyle de 1 à 3 atomes de carbone linéaire ou ramifié
**caractérisée en ce que** le composé de formule générale (II) est couplé avec un composé de formule générale (3)

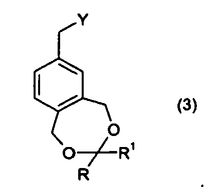

27. Utilisation des composés de formule générale (II') : 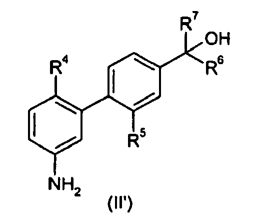 pour la préparation d'analogues non stéroïdiens de vitamine D de formule générale (IV') : 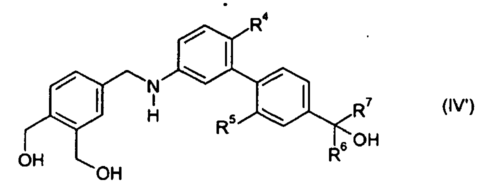 où
- R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis, parmi un radical alkyle linéaire ou ramifié comportant de 1 à 2 atomes de carbone, hydrogéné ou perfluoré
**caractérisée en ce que** le composé de formule générale (II') est couplé avec un composé de formule générale (4)

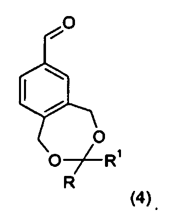

28. Utilisation des composés de formule générale (II") 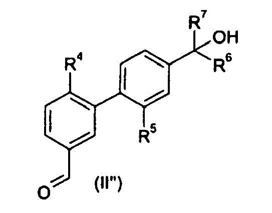 pour la préparation d'analogues non stéroïdiens de vitamine D de formule générale (IV") : 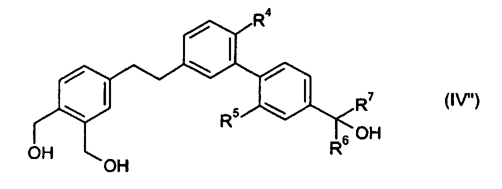 où
- R⁴ et R⁵, identiques ou différents, représentent indépendamment les uns des autres un radical alkyle linéaire ou ramifié comportant de 1 à 3 atomes de carbone ;
- R⁶ et R⁷, identiques, sont choisis parmi un radical alkyle comportant de 1 à 2 atomes de carbone, hydrogéné ou perfluoré
**caractérisé en ce que** le composé de formule générale (II") est couplé avec un composé de formule générale (5)

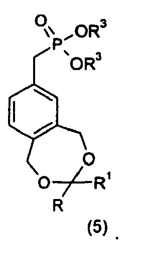

## Claims

1. 7,7-Disubstituted (5*H*,9*H*)-6,8-dioxabenzocycloheptene compound of general formula (I): 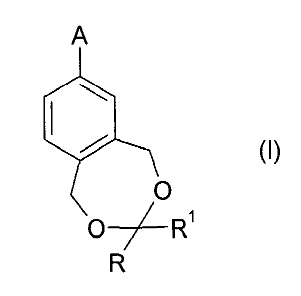 in which:
- A represents a group -CH₂-Y or a carboxaldehyde group
- C(=O)H;
- Y is chosen from a halogen atom, such as chlorine or bromine, a hydroxyl radical, a radical -OSO₂R², and a radical P(O)(OR³)₂;
- R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro;
- it being also possible for R and R¹ to form together and with the carbon atom carrying them a ring of 5 or 6 carbon atoms;
- R² represents an alkyl radical or an aryl radical; and
- R³ represents an alkyl radical;
it being understood that R and R¹ cannot simultaneously be a methyl radical when A is the radical -CH2OH
and their optical isomers.

2. Compound according to Claim 1, **characterized in that** at least one of R or R¹ does not represent hydrogen.

3. Compound according to Claim 1 or 2, **characterized in that** the alkyl radicals having from 1 to 4 carbon atoms are chosen from methyl, ethyl, propyl, isopropyl, 2-methylpropyl, butyl, sec-butyl and tert-butyl radicals.

4. Compound according to any one of the preceding claims, **characterized in that** the alkoxy radicals are chosen from methoxy, ethoxy, propoxy, isopropoxy, butoxy, sec-butoxy and tert-butoxy radicals.

5. Compound according to any one of the preceding claims, **characterized in that** the aryl radicals are chosen from phenyl, naphthyl and pyridyl.

6. Compound according to any one of the preceding claims, **characterized in that** R and R¹ form together and with the carbon atom carrying them a ring of 5 or 6 carbon atoms chosen from a cyclopentane radical and a cyclohexane radical.

7. Compound according to any one of Claims 1 to 5, **characterized in that** A represents a group -CH₂-Y, Y represents a chlorine or bromine atom or a radical OSO₂R² or a radical P(O)(OR³)₂, and R represents a hydrogen atom and R¹ represents a nitrophenyl radical or conversely for R and R¹.

8. Compound according to any one of Claims 1 to 5,
**characterized in that** A represents a group -CH₂-Y, Y represents a hydroxyl radical, and R represents a hydrogen atom and R¹ represents a nitrophenyl radical or conversely for R and R¹.

9. Method for preparing the compounds of formula (I): 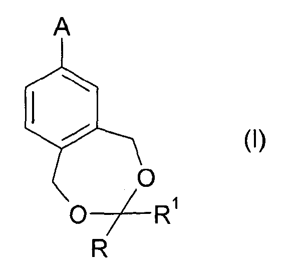 in which:
- A represents a group -CH₂-Y or a carboxaldehyde group
- C(=O)H;
- Y is chosen from a halogen atom, such as chlorine or bromine, a hydroxyl radical, a radical -OSO₂R², and a radical P(O)(OR³)₂;
- R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro;
- it being also possible for R and R¹ to form together and with the carbon atom carrying them a ring of 5 or 6 carbon atoms;
- R² represents an alkyl radical or an aryl radical; and
- R³ represents an alkyl radical;
**characterized in that**:
- in step 1, a trialkyl ester of 1,2,4-benzenetricarboxylic acid is subjected to a reduction reaction, in an aprotic solvent, to give (3,4-bis-hydroxymethylphenyl)methanol,
- in step 2, the said (3,4-bis-hydroxymethylphenyl)methanol is subjected to a reaction for selective protection of the two adjacent benzyl alcohol functional groups at the 3 and 4 positions by reaction with a ketone or an aldehyde of formula R-CO-R¹ in an aprotic solvent, in the presence of a catalyst, to give the compounds of general formula (2): 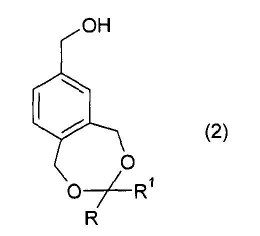
- in step 3(a), the compounds of formula (2) are converted, by reaction with a derivative R²SO₂Cl, to compounds of formula (3): 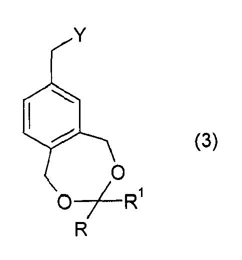 in which Y represents a halogen atom, chosen from a chlorine or bromine atom, or a group -OSO₂R²,
- in step 4, the compounds of formula (3) are converted to compounds of formula (5): 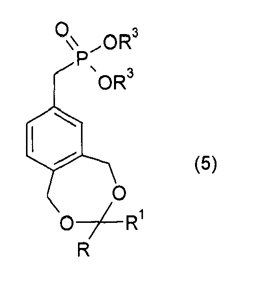 by reaction with a trialkylphosphite derivative P(OR³)₃.

10. Method for preparing the compounds of formula (I): 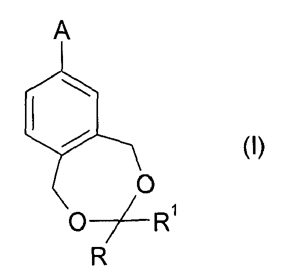 in which:
- A represents a group -CH₂-Y or a carboxaldehyde group
- C (=O) H;
- Y is chosen from a halogen atom, such as chlorine or bromine, a hydroxyl radical, a radical -OSO₂R², and a radical P (O) (OR³)₂;
- R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro;
- it being also possible for R and R¹ to form together and with the carbon atom carrying them a ring of 5 or 6 carbon atoms;
- R² represents an alkyl radical or an aryl radical; and
- R³ represents an alkyl radical;
**characterized in that**:
- in step 1, a trialkyl ester of 1,2,4-benzenetricarboxylic acid is subjected to a reduction reaction, in an aprotic solvent, to give (3,4-bis-hydroxymethylphenyl)methanol,
- in step 2, the said (3,4-bis-hydroxymethylphenyl)methanol is subjected to a reaction for selective protection of the two adjacent benzyl alcohol functional groups at the 3 and 4 positions by reaction with a ketone or an aldehyde of formula R-CO-R¹ in an aprotic solvent, in the presence of a catalyst, to give the compounds of general formula (2): 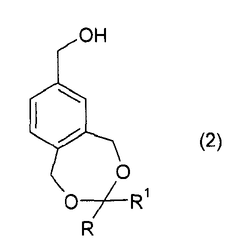
- in step 3(b), the compounds of formula (2) are used in an oxidation reaction, by the action of an oxidizing agent, to give the compounds of formula (4):

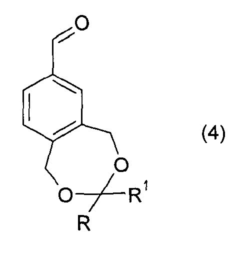

11. Compound of the following formula (2): 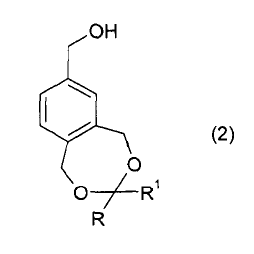 in which R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro, it being also possible for R and R¹ to form together and with the carbon atom carrying them a ring of 5 or 6 carbon atoms provided that R and R¹ are not simultaneously a methyl radical.

12. Compounds of the following formula (3): 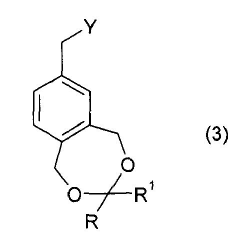 in which:
- Y represents a halogen atom, chosen from chlorine or bromine, or a group -OSO₂R²,
- R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro, it being also possible for R and R¹ to form together and with the carbon atom carrying them a ring of 5 or 6 carbon atoms, and
- R² represents an alkyl radical or an aryl radical.

13. Compounds of the following formula (4): 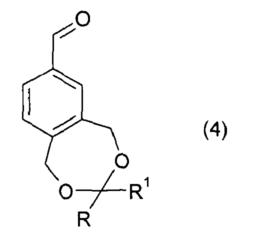 in which R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro, it being also possible for R and R¹ to form together and with the carbon atom carrying them a ring of 5 or 6 carbon atoms.

14. Compounds of the following formula (5): 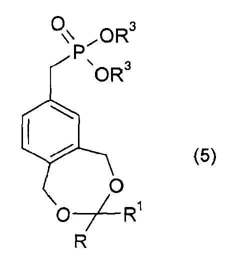 in which R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro, it being also possible for R and R¹ to form together and with the carbon atom carrying them a ring of 5 or 6 carbon atoms and R³ represents an alkyl radical.

15. Use of the compounds of general formula (I): 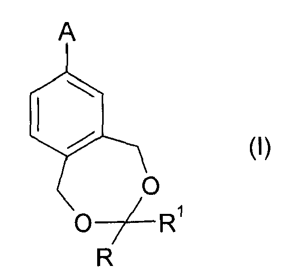 in which:
- A represents a group -CH₂-Y or a carboxaldehyde group
- C (=O) H;
- Y is chosen from a halogen atom, chosen from chlorine or bromine, a hydroxyl radical, a radical -OSO₂R², and a radical P(O)(OR³)₂;
- R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro;
- it being also possible for R and R¹ to form together and with the carbon atom carrying them a ring of 5 or 6 carbon atoms;
- R² represents an alkyl radical or an aryl radical; and
- R³ represents an alkyl radical;
their optional optical isomers,
for the synthesis of non-steroidal analogues of vitamin D.

16. Use according to Claim 15, **characterized in that** the compound of general formula (I) is a compound of the following formula (2):

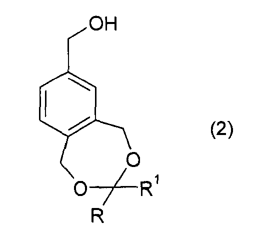

17. Use according to Claim 15, **characterized in that** the compound of general formula (I) is a compound of the following formula (3): 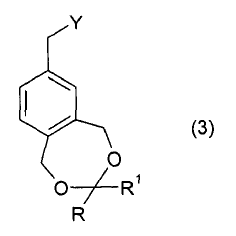 in which Y represents a halogen atom, chosen from chlorine or bromine, or a group -OSO₂R².

18. Use according to Claim 15, **characterized in that** the compound of general formula (I) is a compound of the following formula (4):

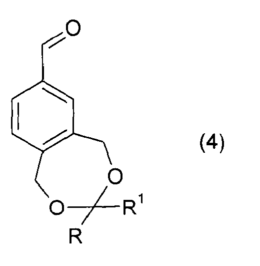

19. Use according to Claim 15, **characterized in that** the compound of general formula (I) is a compound of the following formula (5):

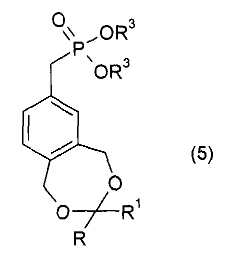

20. Use according to any one of Claims 15 to 19 for the synthesis of non-steroidal analogues of vitamin D of the following formula (IV): 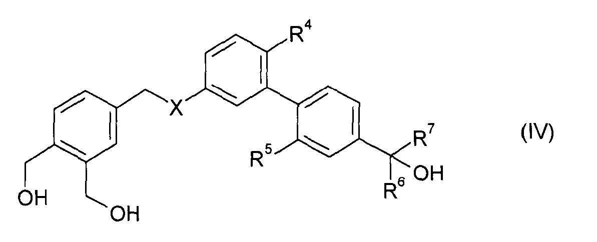 in which:
- R⁴ and R⁵, which are identical or different, represent, independently of each other, a linear or branched alkyl radical comprising from 1 to 3 carbon atoms;
- R⁶ and R⁷, which are identical, are chosen from a linear or branched alkyl radical comprising from 1 to 2 carbon atoms, hydrogenated or perfluorinated;
- X represents -CH₂-, -NH-, -NR⁸- or -O- and
- R⁸ represents a linear or branched alkyl radical of 1 to 3 carbon atoms.

21. Method for the synthesis of non-steroidal analogues of vitamin D, **characterized in that** at least one step uses the compounds of the following general formula (I): 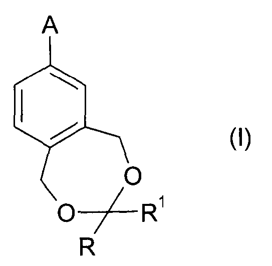 in which:
- A represents a group -CH₂-Y or a carboxaldehyde group
- C (=O) H;
- Y is chosen from a halogen atom, chosen from chlorine or bromine, a hydroxyl radical, a radical -OSO₂R², and a radical P(O) (OR³)₂;
- R and R¹, which are identical or different, are chosen, independently of each other, from a hydrogen atom, an alkyl radical, an alkoxy radical, an aryl radical optionally substituted with one, two, three, four or five substituents chosen from a halogen atom, the halogen being chosen from a chlorine, bromine, iodine or fluorine atom, an alkyl radical, an alkoxy radical, a cyano and a nitro;
- it being also possible for R and R¹ to form together and with the carbon atom carrying them a ring of 5 or 6 carbon atoms;
- R² represents an alkyl radical or an aryl radical; and
- R³ represents an alkyl radical;
and their optical isomers.

22. Method according to Claim 21, **characterized in that** the non-steroidal analogues of vitamin D are the compounds of the following general formula (IV): 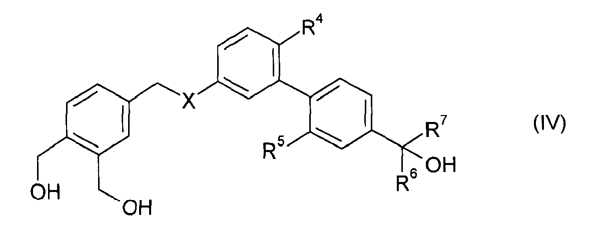 in which:
- R⁴ and R⁵, which are identical or different, represent, independently of each other, a linear or branched alkyl radical comprising from 1 to 3 carbon atoms;
- R⁶ and R⁷, which are identical, are chosen from an alkyl radical comprising from 1 to 2 carbon atoms, hydrogenated or perfluorinated;
- X represents -CH₂-, -NH-, -NR⁸- or -O- and
- R⁸ represents a linear or branched alkyl radical of 1 to 3 carbon atoms.

23. Method according to either of Claims 21 and 22,
**characterized in that** it comprises the following steps:
a) reaction for coupling of the compound of formula (3) of the present invention: 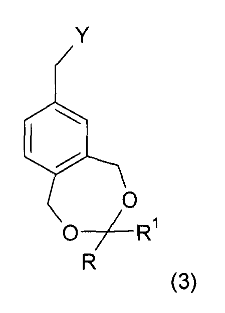 with a compound of formula (II): 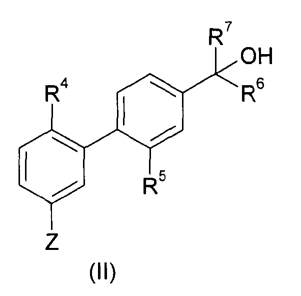 for the production of a compound of formula (III): 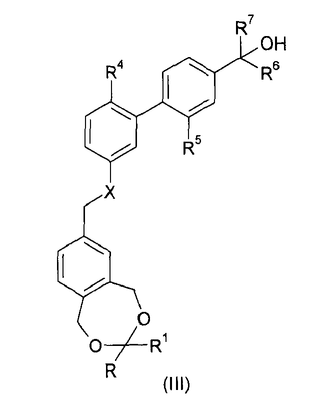
b) reaction for deprotection of the two adjacent benzyl alcohol functional groups at the 3 and 4 positions contained in the (5H,9H)-6,8-dioxabenzocycloheptene ring of the compound of formula (III), the said ring being obtained from the compound of formula (3), for the production of the compound of formula (IV): 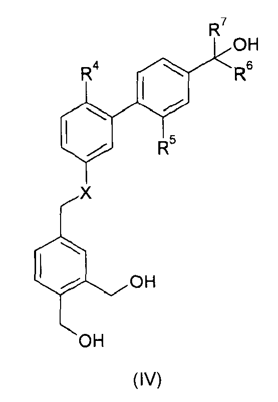 and in which Y represents a halogen atom, chosen from a chlorine or bromine atom, or a radical -OSO₂R², Z represents a hydroxyl radical, or a radical -NHR⁸, R⁸ representing a linear or branched alkyl radical of 1 to 3 carbon atoms.

24. Method according to either of Claims 21 and 22, **characterized in that** it comprises the following steps:
a) coupling reaction between the aldehyde functional group of the compound of formula (4): 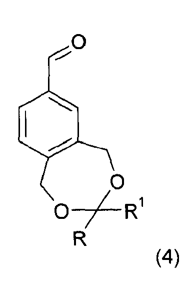 and the amine functional group of the compound of formula (II'): 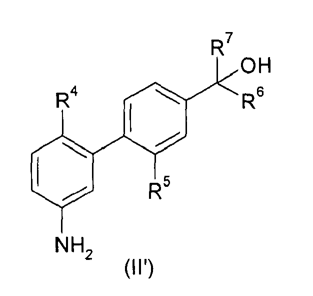 for the production of the compound of formula (III'): 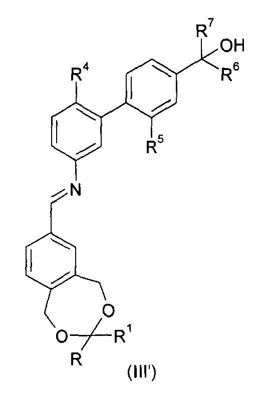
b) hydrogenation and hydrogenolysis reaction on the compound of formula (III'), leading to the reduction of the imine functional group and the deprotection of the two adjacent benzyl alcohol functional groups at the 3 and 4 positions contained in the (5H,9H)-6,8-dioxabenzocycloheptene ring obtained from the compound of formula (4) (hydrogenolysis), for the production of the compound of formula (IV'):

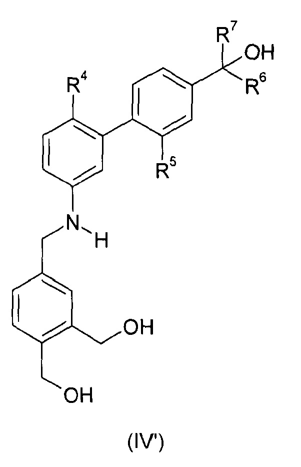

25. Method according to either of Claims 21 and 22, **characterized in that** it comprises the following steps:
a) a so-called Horner-Emmons reaction between the compound of formula (5): 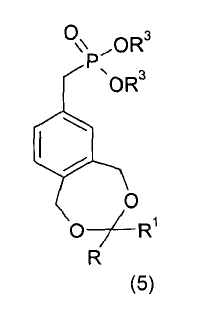 and the compound of formula (II"): 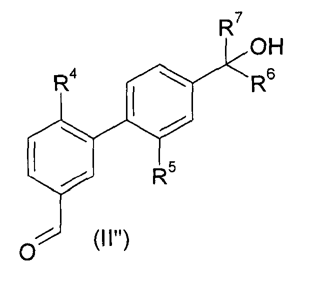 for the production of the compound of formula (III"): 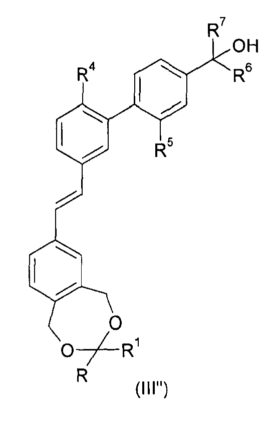
b) hydrogenation and hydrogenolysis reaction on the compound of formula (III"), leading to the reduction of the double bond (hydrogenation) and the deprotection of the two adjacent benzyl alcohol functional groups at the 3 and 4 positions contained in the (5H,9H)-6,8-dioxabenzocycloheptene ring obtained from the compound of formula (5) (hydrogenolysis), for the production of the compound (IV"):

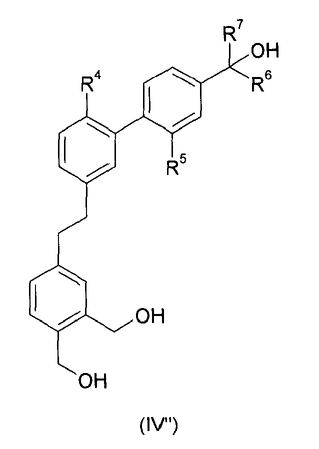

26. Use of the compounds of general formula (II): 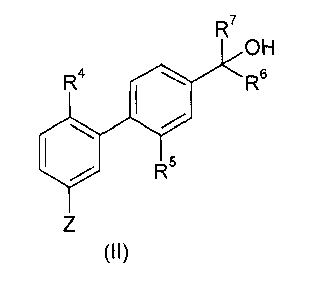 for the preparation of non-steroidal analogues of vitamin D of general formula (IV): 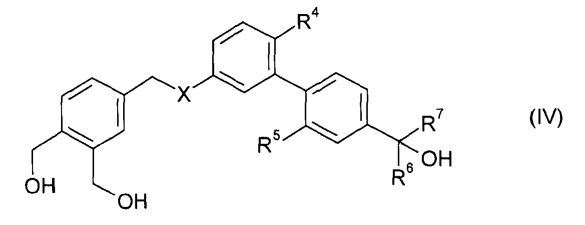 in which:
- R⁴ and R⁵, which are identical or different, represent, independently of each other, a linear or branched alkyl radical comprising from 1 to 3 carbon atoms;
- R⁶ and R⁷, which are identical, are chosen from a linear or branched alkyl radical comprising from 1 to 2 carbon atoms, hydrogenated or perfluorinated;
- X represents -CH₂-, -NH-, -NR⁸- or -O- and
- R⁸ represents a linear or branched alkyl radical of 1 to 3 carbon atoms
**characterized in that** the compound of general formula (II) is coupled with a compound of general formula (3)

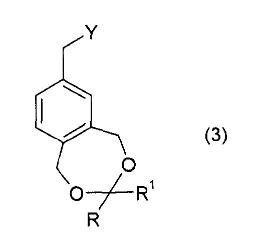

27. Use of the compounds of general formula (II'): 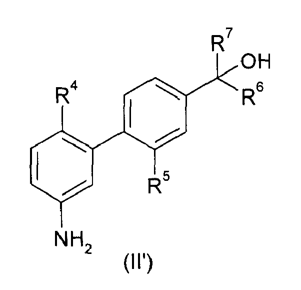 for the preparation of non-steroidal analogues of vitamin D of the general formula (IV'): 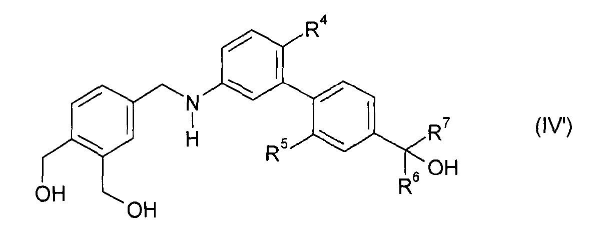 in which:
- R⁴ and R⁵, which are identical or different, represent, independently of each other, a linear or branched alkyl radical comprising from 1 to 3 carbon atoms;
- R⁶ and R⁷, which are identical, are chosen from a linear or branched alkyl radical comprising from 1 to 2 carbon atoms, hydrogenated or perfluorinated
**characterized in that** the compound of general formula (II') is coupled with a compound of general formula (4)

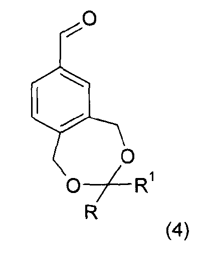

28. Use of the compounds of general formula (II"): 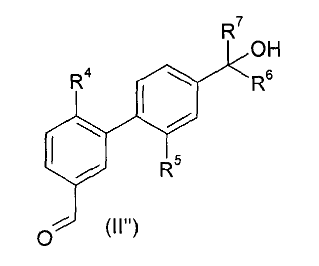 for the preparation of non-steroidal analogues of vitamin D of general formula (IV"): 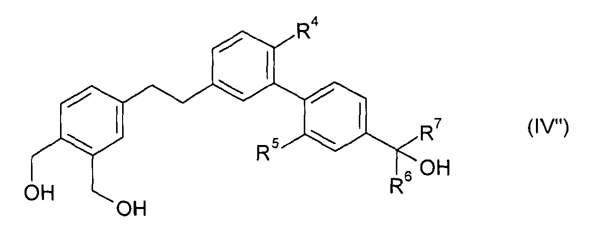 in which:
- R⁴ and R⁵, which are identical or different, represent, independently of each other, a linear or branched alkyl radical comprising from 1 to 3 carbon atoms;
- R⁶ and R⁷, which are identical, are chosen from an alkyl radical comprising from 1 to 2 carbon atoms, hydrogenated or perfluorinated
**characterized in that** the compound of general formula (II") is coupled with a compound of general formula (5)

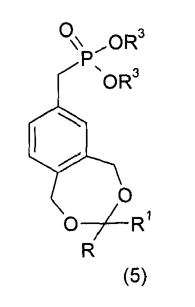

## Patentansprüche

1. 7,7-Disubstituiertes (5H,9H)-6,8-Dioxabenzocyclohepten der allgemeinen Formel (I): 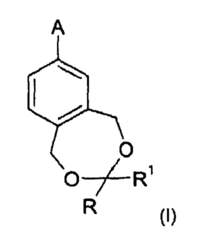 in der Formel:
• A bedeutet eine Gruppe -CH₂-Y oder eine Carboxaldehydgruppe -C(=O)H;
• Y ist ausgewählt unter einem Halogenatom, wie Chlor oder Brom, einer Hydroxygruppe, einer Gruppe -OSO₂R² und einer Gruppe P(O)(OR³)₂;
• R und R¹, die gleich oder verschieden sind, sind unabhängig voneinander ausgewählt unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe;
• R und R¹ können auch gemeinsam und mit dem Kohlenstoffatom, das sie trägt, einen Ring mit 5 oder 6 Kohlenstoffatomen bilden;
• R² bedeutet eine Alkylgruppe oder eine Arylgruppe; und
• R³ bedeutet eine Alkylgruppe,
• mit der Maßgabe, dass R und R¹ nicht gleichzeitig eine Methylgruppe bedeuten können, wenn A die Gruppe -CH₂OH ist;
und ihre optischen Isomere.

2. Verbindung nach Anspruch 1, **dadurch gekennzeichnet, dass** mindestens eine der Gruppen R oder R¹ nicht Wasserstoff bedeutet.

3. Verbindung nach Anspruch 1 oder 2, **dadurch gekennzeichnet, dass** die Alkylgruppen, die 1 bis 4 Kohlenstoffatome aufweisen, unter Methyl, Ethyl, Propyl, Isopropyl, 2-Methylpropyl, Butyl, sek-Butyl und tert-Butyl ausgewählt sind.

4. Verbindung nach einem der vorhergehenden Ansprüche, **dadurch gekennzeichnet, dass** die Alkoxygruppen unter Metho, Ethoxy, Propoxy, Isopropoxy, Butoxy, *sek*-Butoxy und *tert*-Butoxy ausgewählt sind.

5. Verbindung nach einem der vorhergehenden Ansprüche, **dadurch gekennzeichnet, dass** die Arylgruppen unter Phenyl, Naphthyl und Pyridyl ausgewählt sind.

6. Verbindung nach einem der vorhergehenden Ansprüche, **dadurch gekennzeichnet, dass** die Gruppen R und R¹ gemeinsam und mit dem Kohlenstoffatom, das sie trägt, einen Ring mit 5 oder 6 Kohlenstoffatomen bilden, der unter einer Cyclopentangruppe und einer Cyclohexangruppe ausgewählt ist.

7. Verbindung nach einem der Ansprüche 1 bis 5, **dadurch gekennzeichnet, dass** A eine Gruppe -CH₂Y- bedeutet, Y ein Chloratom oder ein Bromatom oder eine Gruppe -OSO₂R² oder eine Gruppe P(O)(OR³)₂ ist und R ein Wasserstoffatom bedeutet und R¹ eine Nitrophenylgruppe ist oder die umgekehrten Bedeutungen für R und R¹.

8. Verbindung nach einem der Ansprüche 1 bis 5, **dadurch gekennzeichnet, dass** A eine Gruppe -CH₂Y- bedeutet, Y eine Hydroxygruppe ist und R ein Wasserstoffatom bedeutet und R¹ eine Nitrophenylgruppe ist oder die umgekehrten Bedeutungen für R und R¹.

9. Verfahren zur Herstellung der Verbindungen der Formel (I): 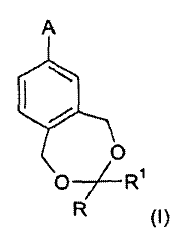 worin:
• A bedeutet eine Gruppe -CH₂-Y oder eine Carboxaldehydgruppe -C(=O)H;
• Y ist ausgewählt unter einem Halogenatom, wie Chlor oder Brom, einer Hydroxygruppe, einer Gruppe -OSO₂R² und einer Gruppe P(O)(OR³)₂;
• R und R¹, die gleich oder verschieden sind, sind unabhängig voneinander ausgewählt unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe;
• R und R¹ können auch gemeinsam und mit dem Kohlenstoffatom, das sie trägt, einen Ring mit 5 oder 6 Kohlenstoffatomen bilden;
• R² bedeutet eine Alkylgruppe oder eine Arylgruppe; und
• R³ bedeutet eine Alkylgruppe,
**dadurch gekennzeichnet, dass**
- in Schritt 1 ein Trialkylester der Benzol-1,2,4-tricarbonsäure in einem aprotischen Lösemittel zur Bildung von (3,4-Bis-hydroxymethylphenyl)methanol einer Reduktion unterzogen wird,
- in Schritt 2 das (3,4-Bis-hydroxymethylphenyl)methanol an den beiden benachbarten Benzylalkoholfunktionen in 3- und 4-Stellung durch Umsetzung mit einem Keton oder Aldehyden der Formel R-CO-R¹ in einem aprotischen Lösemittel in Gegenwart eines Katalysators zur Bildung der Verbindungen der allgemeinen Formel (2) selektiv geschützt wird: 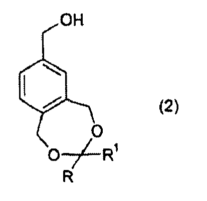
- in Schritt 3(a) die Verbindungen der Formel (2) durch Umsetzung mit einem Derivat R²SO₂Cl in die Verbindungen der Formel (3) umgewandelt werden: 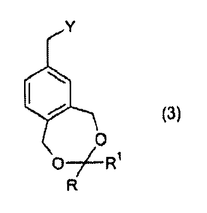 wobei Y ein Halogenatom, das unter einem Chloratom oder einem Bromatom ausgewählt ist, oder eine Gruppe -OSO₂R² bedeutet,
- in Schritt 4 die Verbindungen der Formel (3) in Verbindungen der Formel (5) umgewandelt werden: 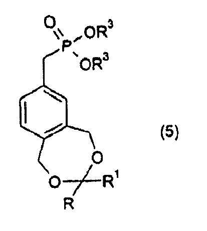 indem sie mit einem Trialkylphosphitderivat P(OR³)₃ umgesetzt werden.

10. Verfahren zur Herstellung der Verbindungen der Formel (I): 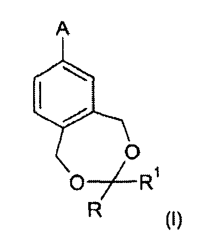 worin:
• A bedeutet eine Gruppe -CH₂-Y oder eine Carboxaldehydgruppe -C(=O)H;
• Y ist ausgewählt unter einem Halogenatom, wie Chlor oder Brom, einer Hydroxygruppe, einer Gruppe -OSO₂R² und einer Gruppe P(O)(OR³)₂;
• R und R¹, die gleich oder verschieden sind, sind unabhängig voneinander ausgewählt unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe;
• R und R¹ können auch gemeinsam und mit dem Kohlenstoffatom, das sie trägt, einen Ring mit 5 oder 6 Kohlenstoffatomen bilden;
• R² bedeutet eine Alkylgruppe oder eine Arylgruppe; und
• R³ bedeutet eine Alkylgruppe,
**dadurch gekennzeichnet, dass**
- in Schritt 1 ein Trialkylester der Benzol-1,2,4-tricarbonsäure in einem aprotischen Lösemittel zur Bildung von (3,4-Bis-hydroxymethylphenyl)methanol einer Reduktion unterzogen wird,
- in Schritt 2 das (3,4-Bis-hydroxymethylphenyl)methanol an den beiden benachbarten Benzylalkoholfunktionen in 3- und 4-Stellung durch Umsetzung mit einem Keton oder Aldehyden der Formel R-CO-R¹ in einem aprotischen Lösemittel in Gegenwart eines Katalysators zur Bildung der Verbindungen der allgemeinen Formel (2) selektiv geschützt wird: 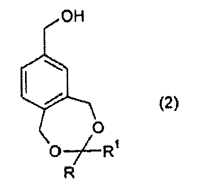
- in Schritt 3(b) die Verbindungen der Formel (2) durch Einwirkung eines Oxidationsmittels zur Bildung der Verbindungen der Formel (4) oxidiert werden:

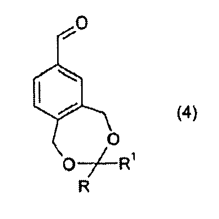

11. Verbindung der folgenden Formel (2): 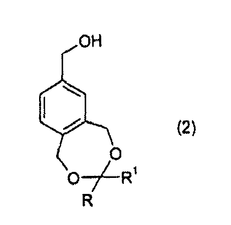 worin die Gruppen R und R¹, die gleich oder verschieden sind, unabhängig voneinander ausgewählt sind unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe, wobei R und R¹ auch gemeinsam und mit dem Kohlenstoffatom, das sie trägt, einen Ring mit 5 oder 6 Kohlenstoffatomen bilden können, mit der Maßgabe, dass R und R¹ nicht gleichzeitig Methyl bedeuten.

12. Verbindungen der folgenden Formel (3) : 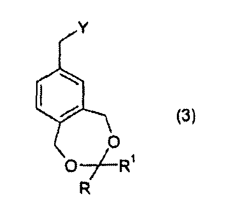 in der Formel:
• Y bedeutet ein Halogenatom, das unter Chlor und Brom ausgewählt ist, oder eine Gruppe -OSO₂R²;
• R und R¹, die gleich oder verschieden sind, sind unabhängig voneinander ausgewählt unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe; wobei R und R¹ auch gemeinsam und mit dem Kohlenstoffatom, das sie trägt, einen Ring mit 5 oder 6 Kohlenstoffatomen bilden können; und
• R² bedeutet eine Alkylgruppe oder eine Arylgruppe.

13. Verbindungen der folgenden Formel (4): 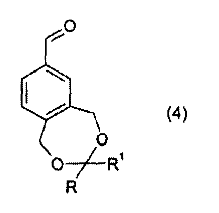 worin die Gruppen R und R¹, die gleich oder verschieden sind, unabhängig voneinander ausgewählt sind unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe, wobei R und R¹ auch gemeinsam und mit dem Kohlenstoffatom, das sie trägt, einen Ring mit 5 oder 6 Kohlenstoffatomen bilden können.

14. Verbindungen der folgenden Formel (5): 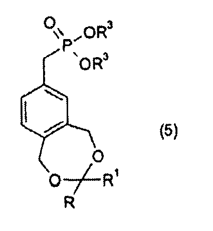 worin die Gruppen R und R¹, die gleich oder verschieden sind, unabhängig voneinander ausgewählt sind unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe, wobei R und R¹ auch gemeinsam und mit dem Kohlenstoffatom, das sie trägt, einen Ring mit 5 oder 6 Kohlenstoffatomen bilden können, und R³ eine Alkylgruppe bedeutet.

15. Verwendung der Verbindungen der allgemeinen Formel (I): 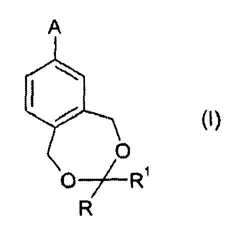 in der Formel:
• A bedeutet eine Gruppe -CH₂-Y oder eine Carboxaldehydgruppe -C(=O)H;
• Y ist ausgewählt unter einem Halogenatom, wie Chlor oder Brom, einer Hydroxygruppe, einer Gruppe -OSO₂R² und einer Gruppe P(O)(OR³)₂;
• R und R¹, die gleich oder verschieden sind, sind unabhängig voneinander ausgewählt unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe;
• R und R¹ können auch gemeinsam und mit dem Kohlenstoffatom, das sie trägt, einen Ring mit 5 oder 6 Kohlenstoffatomen bilden;
• R² bedeutet eine Alkylgruppe oder eine Arylgruppe; und
• R³ bedeutet eine Alkylgruppe,
ihrer möglichen optischen Isomere
für die Synthese von nichtsteroidalen Vitamin D-Analoga.

16. Verwendung nach Anspruch 15, **dadurch gekennzeichnet, dass** die Verbindung der allgemeinen Formel (I) eine Verbindung der folgenden Formel (2) ist:

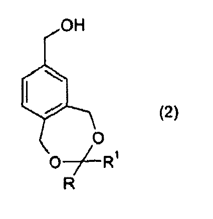

17. Verwendung nach Anspruch 15, **dadurch gekennzeichnet, dass** die Verbindung der allgemeinen Formel (I) eine Verbindung der folgenden Formel (3) ist: 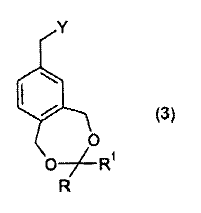 wobei Y ein Halogenatom, das unter einem Chloratom oder einem Bromatom ausgewählt ist, oder eine Gruppe -OSO₂R² bedeutet.

18. Verwendung nach Anspruch 15, **dadurch gekennzeichnet, dass** die Verbindung der allgemeinen Formel (I) eine Verbindung der folgenden Formel (4) ist:

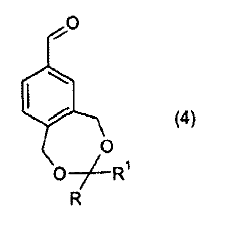

19. Verwendung nach Anspruch 15, **dadurch gekennzeichnet, dass** die Verbindung der allgemeinen Formel (I) eine Verbindung der folgenden Formel (5) ist:

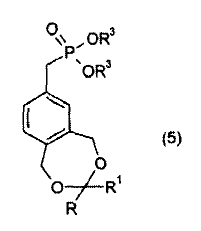

20. Verwendung nach einem der Ansprüche 15 bis 19 zur Synthese von nichtsteroidalen Vitamin D-Analoga der folgenden Formel (IV): 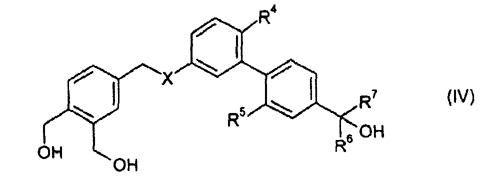 in der Formel:
- R⁴ und R⁵, die gleich oder verschieden sind, bedeuten unabhängig voneinander eine lineare oder verzweigte Alkylgruppe mit 1 bis 3 Kohlenstoffatomen;
- R⁶ und R⁷, die gleich sind, sind unter einer linearen oder verzweigten Alkylgruppe mit 1 bis 2 Kohlenstoffatomen ausgewählt, die hydriert oder perfluoriert ist;
- X bedeutet -CH₂-, -NH-, -NR⁸- oder -O- ; und
- R⁸ ist eine lineare oder verzweigte Alkylgruppe mit 1 bis 3 Kohlenstoffatomen.

21. Verfahren zur Synthese von nichtsteroidalen Vitamin D-Analoga,
**dadurch gekennzeichnet, dass** in zumindest einem Schritt Verbindungen der folgenden allgemeinen Formel (I) eingesetzt werden: 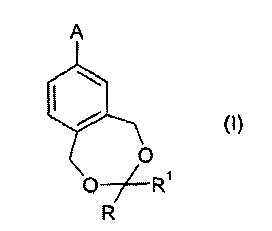 in der Formel:
• A bedeutet eine Gruppe -CH₂-Y oder eine Carboxaldehydgruppe -C(=O)H;
• Y ist ausgewählt unter einem Halogenatom, wie Chlor oder Brom, einer Hydroxygruppe, einer Gruppe -OSO₂R² und einer Gruppe P(O)(OR³)₂;
• R und R¹, die gleich oder verschieden sind, sind unabhängig voneinander ausgewählt unter einem Wasserstoffatom, einer Alkylgruppe, einer Alkoxygruppe, einer Arylgruppe, die gegebenenfalls mit einem, zwei, drei, vier oder fünf Substituenten substituiert ist, die ausgewählt sind unter einem Halogenatom, wobei das Halogenatom unter einem Chloratom, einem Bromatom, einem Iodatom oder einem Fluoratom ausgewählt ist, einer Alkylgruppe, einer Alkoxygruppe, einer Cyanogruppe und einer Nitrogruppe;
• R und R¹ können auch gemeinsam und mit dem Kohlenstoffatom, das sie trägt, einen Ring mit 5 oder 6 Kohlenstoffatomen bilden;
• R² bedeutet eine Alkylgruppe oder eine Arylgruppe; und
• R³ bedeutet eine Alkylgruppe.
sowie ihre optischen Isomere.

22. Verfahren nach Anspruch 21, **dadurch gekennzeichnet, dass** die nicht-steroidalen Vitamin D-Analoga Verbindungen der folgenden Formel (IV) sind: 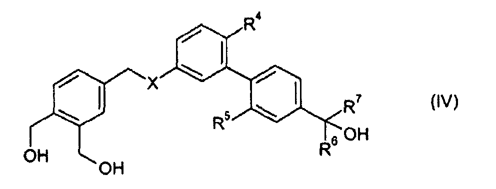 in der Formel:
- R⁴ und R⁵, die gleich oder verschieden sind, bedeuten unabhängig voneinander eine lineare oder verzweigte Alkylgruppe mit 1 bis 3 Kohlenstoffatomen;
- R⁶ und R⁷, die gleich sind, sind unter einer linearen oder verzweigten Alkylgruppe mit 1 bis 2 Kohlenstoffatomen ausgewählt, die hydriert oder perfluoriert ist;
- X bedeutet -CH₂-, -NH-, -NR⁸- oder -O- ; und
- R⁸ ist eine lineare oder verzweigte Alkylgruppe mit 1 bis 3 Kohlenstoffatomen.

23. Verfahren nach einem der Ansprüche 21 und 22, **dadurch gekennzeichnet, dass** es die folgenden Schritte umfasst:
a) Kupplung der erfindungsgemäßen Verbindung der Formel (3): 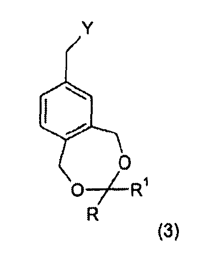 mit einer Verbindung 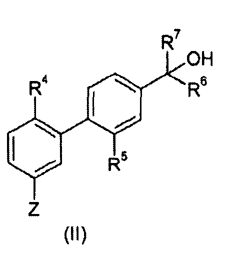 zur Bildung einer Verbindung der Formel (III): 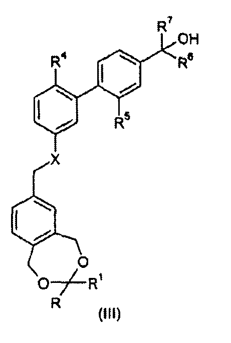
b) Entfernen der Schutzgruppe an den beiden benachbarten Benzylalkoholfunktionen in 3- und 4-Stellung, die an dem (5H,9H)-6,8-Dioxabenzocycloheptenring der Verbindung der Formel (III) vorliegen, wobei der Ring von der Verbindung der Formel (3) stammt, zur Bildung der Verbindung der Formel (IV): 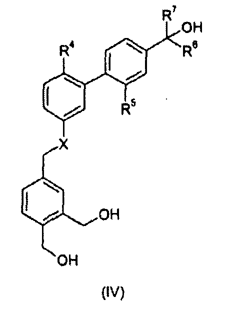
wobei Y ein Halogenatom, das unter einem Chloratom oder einem Bromatom ausgewählt ist, oder eine Gruppe -OSO₂R² bedeutet, Z eine Hydroxygruppe oder eine Gruppe -NHR⁸ ist, wobei R⁸ eine geradkettige oder verzweigte Alkylgruppe mit 1 bis 3 Kohlenstoffatomen bedeutet.

24. Verfahren nach einem der Ansprüche 21 und 22, **dadurch gekennzeichnet, dass** es die folgenden Schritte umfasst:
a) Kupplung der Aldehydfunktion der Verbindung der Formel (4): 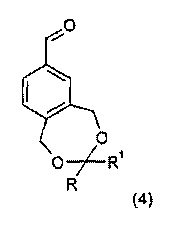 mit der Aminfunktion der Verbindung der Formel (II'): 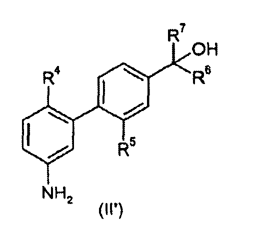 zur Bildung der Verbindung der Formel (III'): 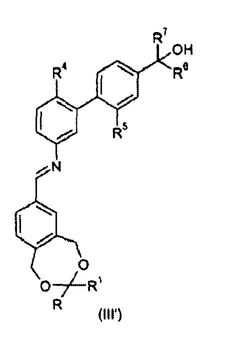
b) Hydrierung und Hydrogenolyse an der Verbindung der Formel (III'), die zur Reduktion der Iminfunktion und zum Entfernen der Schutzgruppe an den beiden benachbarten Benzylalkoholfunktionen in 3- und 4-Stellung führen, die an dem (5H,9H)-6,8-Dioxabenzocycloheptenring vorliegen, der von der Verbindung der Formel (4) stammt (Hydrogenolyse), zur Bildung der Verbindung der Formel (IV'):

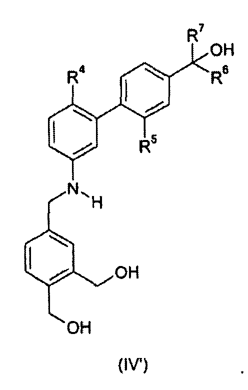

25. Verfahren nach einem der Ansprüche 21 und 22, **dadurch gekennzeichnet, dass** es die folgenden Schritte umfasst:
a) so genannte Horner-Emmons-Reaktion zwischen der Verbindung der Formel (5): 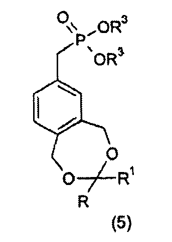 und der Verbindung der Formel (II'): 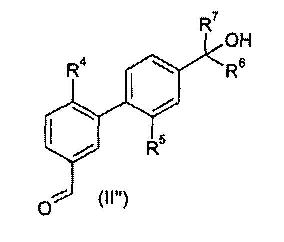 zur Bildung der Verbindung der Formel (III'): 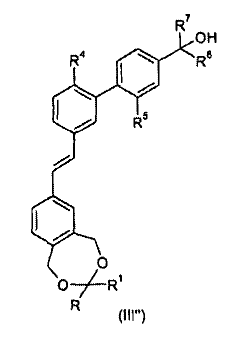
b) Hydrierung und Hydrogenolyse an der Verbindung der Formel (III'), die zur Reduktion der Doppelbindung (Hydrierung) und zum Entfernen der Schutzgruppe an den beiden benachbarten Benzylalkoholfunktionen in 3- und 4-Stellung führen, die an dem (5H,9H)-6,8-Dioxabenzocycloheptenring vorliegen, der von der Verbindung der Formel (5) stammt (Hydrogenolyse), zur Bildung der Verbindung der Formel (IV'):

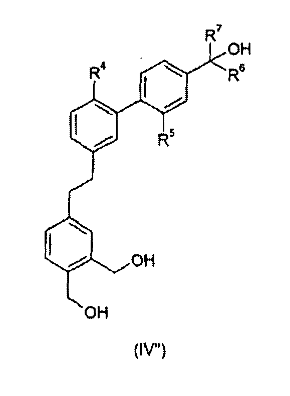

26. Verwendung der Verbindungen der allgemeinen Formel (II): 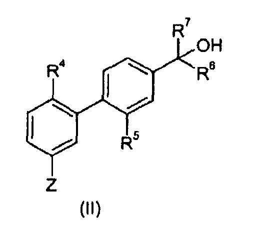 für die Herstellung von nichtsteroidalen Vitamin D-Analoga der folgenden Formel (IV): 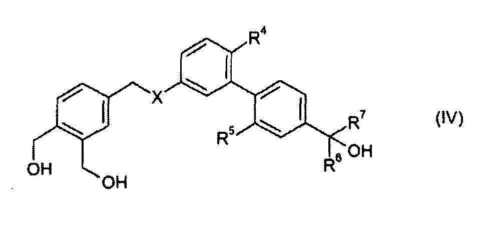 in der Formel:
- R⁴ und R⁵, die gleich oder verschieden sind, bedeuten unabhängig voneinander eine lineare oder verzweigte Alkylgruppe mit 1 bis 3 Kohlenstoffatomen;
- R⁶ und R⁷, die gleich sind, sind unter einer linearen oder verzweigten Alkylgruppe mit 1 bis 2 Kohlenstoffatomen ausgewählt, die hydriert oder perfluoriert ist;
- X bedeutet -CH₂-, -NH-, -NR⁸- oder -O- ; und
- R⁸ ist eine lineare oder verzweigte Alkylgruppe mit 1 bis 3 Kohlenstoffatomen,
**dadurch gekennzeichnet, dass** die Verbindung der allgemeinen Formel (II) mit einer Verbindung der allgemeinen Formel (3) gekuppelt wird:

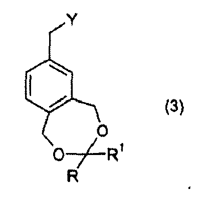

27. Verwendung der Verbindungen der allgemeinen Formel (II'): 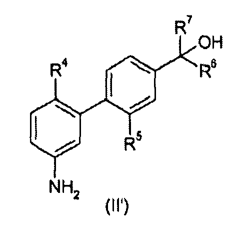 für die Herstellung von nichtsteroidalen Vitamin D-Analoga der folgenden Formel (IV'): 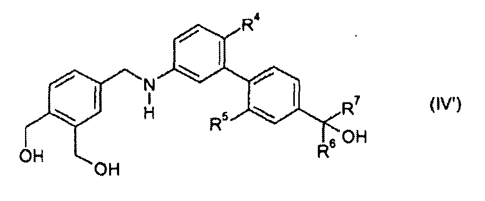 in der Formel:
- R⁴ und R⁵, die gleich oder verschieden sind, bedeuten unabhängig voneinander eine lineare oder verzweigte Alkylgruppe mit 1 bis 3 Kohlenstoffatomen;
- R⁶ und R⁷, die gleich sind, sind unter einer linearen oder verzweigten Alkylgruppe mit 1 bis 2 Kohlenstoffatomen ausgewählt, die hydriert oder perfluoriert ist;
**dadurch gekennzeichnet, dass** die Verbindung der allgemeinen Formel (II') mit einer Verbindung der allgemeinen Formel (4) gekuppelt wird:

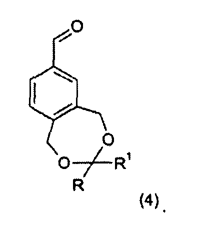

28. Verwendung der Verbindungen der allgemeinen Formel (II'): 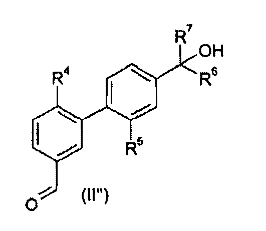 für die Herstellung von nichtsteroidalen Vitamin D-Analoga der folgenden allgemeinen Formel (IV"): 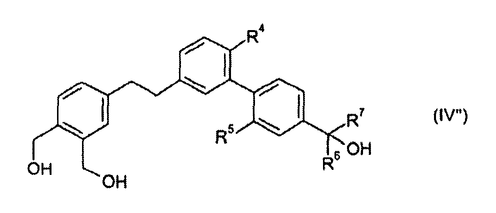 in der Formel:
- R⁴ und R⁵, die gleich oder verschieden sind, bedeuten unabhängig voneinander eine lineare oder verzweigte Alkylgruppe mit 1 bis 3 Kohlenstoffatomen;
- R⁶ und R⁷, die gleich sind, sind unter einer linearen oder verzweigten Alkylgruppe mit 1 bis 2 Kohlenstoffatomen ausgewählt, die hydriert oder perfluoriert ist;
**dadurch gekennzeichnet, dass** die Verbindung der allgemeinen Formel (II') mit einer Verbindung der allgemeinen Formel (5) gekuppelt wird:
